(19) Europäisches Patentamt
European Patent Office
Office européen des brevets

(11) EP 2 899 274 A1

(12) EUROPEAN PATENT APPLICATION
published in accordance with Art. 153(4) EPC

(43) Date of publication:
29.07.2015 Bulletin 2015/31

(21) Application number: 13839049.7

(22) Date of filing: 19.09.2013

(51) Int Cl.:
*C12N 15/09* (2006.01) *C12Q 1/04* (2006.01)
*C12Q 1/68* (2006.01)

(86) International application number:
PCT/JP2013/075335

(87) International publication number:
WO 2014/046198 (27.03.2014 Gazette 2014/13)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 19.09.2012 JP 2012205756
29.05.2013 JP 2013113430

(71) Applicants:
- Sysmex Corporation
  Kobe-shi, Hyogo 651-0073 (JP)
- The University of Tokyo
  Tokyo 113-8654 (JP)

(72) Inventors:
- SAKAI, Ayako
  Kobe-shi
  Hyogo 651-0073 (JP)
- NAGAE, Genta
  Tokyo 113-8654 (JP)
- MIDORIKAWA, Yutaka
  Tokyo 113-8654 (JP)
- KAJITA, Masahiro
  3700033 Gunma (JP)
- ABURATANI, Hiroyuki
  Tokyo 1138654 (JP)

(74) Representative: Paemen, Liesbet R.J. et al
De Clercq & Partners
Edgard Gevaertdreef 10 a
9830 Sint-Martens-Latem (BE)

# (54) METHOD FOR OBTAINING INFORMATION ABOUT HEPATOCELLULAR CARCINOMA, AND MARKER AND KIT FOR OBTAINING INFORMATION ABOUT HEPATOCELLULAR CARCINOMA

(57) The present invention provides a method for obtaining information on hepatocellular carcinoma of a subject based on the result of analysis of methylation status of a CpG site in a promoter region of at least one gene selected from EFNB2, CCNJ and KCTD 12 in DNA extracted from a biological sample of the subject.



Printed by Jouve, 75001 PARIS (FR)

## Description

### TECHNICAL FIELD

The present invention relates to a method for obtaining information on hepatocellular carcinoma in a subject. The present invention also relates to a marker and a kit used in the method.

### BACKGROUND ART

Hepatocellular carcinoma is a type of primary liver cancer and is a malignant tumor originated from hepatocytes. Hepatocellular carcinoma is almost inactive in the initial stage. However, when subjective symptoms such as abdominal lumps, sudden abdominal pain or jaundice are manifested, the disease has already been developed to severe stages in most cases. Hepatocellular carcinoma is known to be often developed in patients with chronic hepatitis resulting from hepatitis viruses or with hepatic cirrhosis. Thus early detection of hepatocellular carcinoma has been sought by identifying the patients as a high-risk group and proactively examining the patients.

Examination for hepatocellular carcinoma includes measurement of tumor markers in blood. The tumor markers currently used include AFP ($\alpha$-fetoprotein), PIVKA-II (protein induced by vitamin-K absence II) and the like. The examination of the tumor markers is used as screening of patients with hepatocellular carcinoma; however the markers have insufficient sensitivity and specificity. For example, the examination of AFP may not result in an abnormal value for hepatocellular carcinoma with relatively small size and the examination of PIVKA-II may provide false positive for subjects with vitamin K deficiency or subjects taking warfarin. Therefore definitive diagnosis of hepatocellular carcinoma is made by, in addition to the examination of tumor markers, ultrasonic examination and examination by imaging such as CT and MRI.

Meanwhile, new diagnosis methods for cancer have been recently studied which are based on genetic information. The methods include, for example, ones based on information on methylation of DNAs. The methods use markers which are CpG sites (5'-(CG)-3') in base sequences of certain genes. Based on the analysis results of the methylation status of the markers, information such as presence or absence of a cancer cell is obtained, which information may be used as an index for diagnosis of cancer.

Methods for determining cancer utilizing methylation analyses of DNA have been studied and developed for hepatocellular carcinoma. For example, the publication by Lee S. et al. discloses that genes such as GSTP1, p16 (also referred to as CDKN2A) and E-cadherin (also referred to as CDH1) are not methylated in non-cancerous liver tissues while the genes are highly methylated in tissues of hepatocellular carcinoma (see Non-Patent Literature 1).

Citation List

Non-Patent Literature

Non-Patent Literature 1: Lee S. et al., Am. J. Pathol. vol. 163, no. 4, p. 1371-1378 (2003)

### SUMMARY OF THE INVENTION

Although some genes indicative of abnormal methylation in hepatocellular carcinoma have been reported as described above, these genes contain those which are methylated in some extent in a type of cancer different from hepatocellular carcinoma. When methylation analysis is carried out with a marker that is methylated in several types of cancer including hepatocellular carcinoma, information deduced from the analysis result may include not only information on hepatocellular carcinoma but also information on other types of cancer. In this case, even when an analysis result provides information indicating that a sample contains a cancer cell for example, it is difficult to determine whether the cancer cell is derived from hepatocellular carcinoma or from other types of cancer.

As such upon obtaining information on hepatocellular carcinoma by methylation analysis of a marker, the specificity of the marker to hepatocellular carcinoma is very important. Thus there is a need for a novel marker specific to hepatocellular carcinoma, which allows specific detection of a cancer cell derived from hepatocellular carcinoma.

Accordingly an object of the present invention is to provide a method for obtaining information on a cancer cell derived from hepatocellular carcinoma by identifying such a novel marker and analyzing methylation status of the marker. Another object of the present invention is to provide a kit suitably used for the method.

The inventors of the present invention have identified novel markers which are genetic regions specifically methylated in DNAs obtained from cancerous tissues of hepatocellular carcinoma. The inventors of the present invention have found that cancer cells derived from hepatocellular carcinoma can be clearly discriminated from other cells (cells of normal tissues, cells of non-cancerous tissues and cancer cells derived from a type of cancer other than hepatocellular carcinoma) based on the result obtained by analyzing methylation status of the markers, thereby completing the present invention.

Thus the present invention provides a method for obtaining information on hepatocellular carcinoma comprising the steps of:

preparing a DNA sample from a biological sample collected from a subject;
analyzing methylation status of a CpG site in a promoter region of at least one gene selected from EFNB2 (Ephrin-B2), CCNJ (Cyclin J) and KCTD12 (Potassium channel tetramerisation domain containing 12) in the DNA sample obtained from the preparation step; and
obtaining information on hepatocellular carcinoma in the subject based on an analysis result obtained from the analyzing step.

The present invention also provides a marker for obtaining information on hepatocellular carcinoma by methylation analysis, which is at least one CpG site selected from CpG sites located in promoter regions of EFNB2, CCNJ and KCTD12 genes.

The present invention also provides a kit for obtaining information on hepatocellular carcinoma, comprising a primer set for analysis of methylation status of at least one CpG site selected from CpG sites located in promoter regions of EFNB2, CCNJ and KCTD 12 genes.

The present invention can provide a novel marker which allows provision of information on hepatocellular carcinoma. The present invention can also provide a method and a kit for obtaining information on hepatocellular carcinoma in a subject by analyzing methylation status of the marker.

**BRIEF DESCRIPTION OF THE DRAWINGS**

Fig. 1A is a graph showing the methylation positive rate of the promoter region of EFNB2 gene in DNAs extracted from normal liver tissues, cirrhosis tissues and hepatocellular carcinoma tissues;
Fig. 1B is a graph showing the methylation positive rate of the promoter region of CCNJ gene in DNAs extracted from normal liver tissues, cirrhosis tissues and hepatocellular carcinoma tissues;
Fig. 1C is a graph showing the methylation positive rate of the promoter region of KCTD12 gene in DNAs extracted from normal liver tissues, cirrhosis tissues and hepatocellular carcinoma tissues;
Fig. 2A is a graph showing the methylation positive rate of the promoter region of EFNB2 gene calculated from methylation data of various clinical specimens;
Fig. 2B is a graph showing the methylation positive rate of the promoter region of CCNJ gene calculated from methylation data of various clinical specimens;
Fig. 2C is a graph showing the methylation positive rate of the promoter region of KCTD12 gene calculated from methylation data of various clinical specimens;
Fig. 3A is a graph showing the methylation positive rate of the promoter region of a known marker gene GSTP1 calculated from methylation data of various clinical specimens;
Fig. 3B is a graph showing the methylation positive rate of the promoter region of a known marker gene CDKN2A calculated from methylation data of various clinical specimens;
Fig. 3C is a graph showing the methylation positive rate of the promoter region of a known marker gene CDH1 calculated from methylation data of various clinical specimens;
Fig. 4 is a graph showing the methylation score of the promoter region of EFNB2 gene in DNAs extracted from various clinical specimens;
Fig. 5 is an image showing the results of amplification by methylation specific PCR (MSP) of DNAs extracted from normal liver tissues, cirrhosis tissues and hepatocellular carcinoma tissues using the respective primer sets for EFNB2, CCNJ and KCTD12;
Fig. 6 is a graph showing the methylation positive rate of the promoter region of EFNB2 gene calculated from methylation data of normal liver tissues and cancerous tissues of hepatocellular carcinoma and non-cancerous tissues;
Fig. 7 is a graph showing the methylation positive rate of the promoter region of EFNB2 gene calculated from methylation data of various clinical specimens;
Fig. 8A is a graph showing the methylation positive rate of the promoter region of a known marker gene CDH 11 calculated from methylation data of various clinical specimens;
Fig. 8B is a graph showing the methylation positive rate of the promoter region of a known marker gene CDKN2A calculated from methylation data of various clinical specimens;

Fig. 8C is a graph showing the methylation positive rate of the promoter region of a known marker gene GSTP1 calculated from methylation data of various clinical specimens;

Fig. 9 is an image showing the results of amplification by MSP of DNAs extracted from normal liver tissues and cancerous tissues of hepatocellular carcinoma and non-cancerous tissues using a primer set for EFNB2 (SEQ ID NOs: 20 and 21);

Fig. 10A is a bar graph showing the band intensity of amplification products obtained by MSP of DNA extracted from peripheral blood of hepatocellular carcinoma patients and healthy subjects with a primer set for EFNB2 (SEQ ID NOs: 8 and 9);

Fig. 10B is a bar graph showing the band intensity of amplification products obtained by MSP of DNA extracted from peripheral blood of hepatocellular carcinoma patients and healthy subjects with a primer set for CCNJ (SEQ ID NOs: 10 and 11);

Fig. 10C is a bar graph showing the band intensity of amplification products obtained by MSP of DNA extracted from peripheral blood of hepatocellular carcinoma patients and healthy subjects with a primer set for EFNB2 (SEQ ID NOs: 20 and 21);

Fig. 10D is a bar graph showing the band intensity of amplification products obtained by MSP of DNA extracted from peripheral blood of hepatocellular carcinoma patients and healthy subjects with a primer set for accuracy control (SEQ ID NOs: 22 and 23);

Fig. 11 is a schematic view of an example of a determination device for providing information on hepatocellular carcinoma in a subject;

Fig. 12 is a block diagram showing the functionality configuration of the determination device of Fig. 11;

Fig. 13 is a block diagram showing the hardware configuration of the determination device shown in Fig. 11; and

Fig. 14 is a flow chart of determination for providing information on hepatocellular carcinoma in a subject using the determination device in Fig. 11.

## MODES FOR CARRYING OUT THE INVENTION

In the method for obtaining information on hepatocellular carcinoma of the present invention (hereinafter also merely referred to as "method"), a DNA sample is first prepared from a biological sample collected from a subject.

In the present embodiments, the biological sample is not particularly limited as far as it is a biological sample containing DNA of a subject and is preferably a sample containing a genomic DNA such as a clinical specimen. The clinical specimen may include, for example, body fluid, urine, tissues obtained by operations or biopsies and the like. The body fluid may include blood, serum, plasma, lymph fluid, ascetic fluid, bone marrow aspirate, nipple discharge and the like. The biological sample may also be a culture medium obtained by culturing cells or tissues collected from a subject.

The DNA sample can be prepared by extracting DNA from the biological sample. The method for extracting DNA from biological samples is well known in the art. Extraction of DNA can be carried out, for example, by mixing the biological sample with a treatment solution containing a surfactant for solubilization of cells or tissues (e.g. sodium cholate, sodium dodecyl sulfate etc.), and subjecting the resulting mixture to physical procedure (stirring, homogenization, ultrasonication etc.) to release DNA contained in the biological sample into the mixture. In this case, it is preferable to centrifuge the mixture to precipitate cell debris and use the supernatant containing the released DNA to the next step of analyzing. The obtained supernatant may be purified according to well-known methods. DNA can also be extracted and purified from a biological sample by using commercially available kits.

The preparation step preferably further comprises the step of fragmenting the extracted DNA. By fragmenting DNA to have appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. When the restriction enzyme treatment is used for DNA fragmentation, the restriction enzyme may appropriately be selected based on the base sequence of DNA, which may be *Mse*I or B*am*HI, for example.

According to the present method, methylation status of a CpG site in a promoter region of at least one gene selected from EFNB2, CCNJ and KCTD12 in DNA obtained from the preparation step is analyzed.

As used herein, the term "CpG site" means a site of a sequence in which cytosine (C) and guanine (G) are adjacent in this order from 5' to 3'. The letter "p" in "CpG" represents a phosphodiester bond between cytosine and guanine.

As used herein, to "analyze methylation status" means to analyze presence or absence of methylation of a CpG site located in a promoter region of at least one gene selected from EFNB2, CCNJ and KCTD12 or analyze methylation frequency in the promoter region.

The base sequences *per se* of the promoter regions of EFNB2, CCNJ and KCTD 12 genes are well known in the art. The base sequences can be obtained from well-known databases such as the one provided by the National Center for Biotechnology Information (NCBI) (http://www.ncbi.nlm.nih.gov/). The ID numbers of the above genes are shown in Table 1. The base sequences of the promoter regions of the genes are represented by SEQ ID NOs: 1, 2 and 3, respectively (the base sequences SEQ ID NOs: 1 and 2 correspond to the sequences of negative strands and the base sequence SEQ ID NO: 3 corresponds to the sequence of the positive strand).

Table 1

| **Gene symbol** | Unigene ID | Entrez Gene ID | Transcript ID | SEQ ID NO: |
|---|---|---|---|---|
| *EFNB2* | Hs. 149239 | 1948 | NM_004093.3 | 1 |
| CCNJ | Hs.596479 | 54619 | NM_001134375<br>NN_019084<br>NM_001134376 | 2 |
| *KCTD12* | Hs.644125 | 115207 | NM_138444 | 3 |

In the embodiments of the present invention, the analyzing step may be the step of analyzing presence or absence of methylation of at least one CpG site among CpG sites located in a promoter region of at least one gene selected from EFNB2, CCNJ and KCTD12. The term "presence or absence of methylation" means whether or not cytosine in a CpG site located in the promoter region is methylated. In the embodiment, one CpG site may be analyzed; however, more than one CpG site is preferably analyzed for presence or absence of methylation. More than one CpG site may be selected from a promoter region of one gene or from each of promoter regions of more than one gene.

In another embodiment of the present invention, the analyzing step may be the step of analyzing methylation frequency in a promoter region of at least one gene selected from EFNB2, CCNJ and KCTD12. The term "methylation frequency" means the ratio of the number of methylated CpG site(s) relative to the number of CpG site(s) located in the promoter region. In the embodiment, the target for analysis may be the whole promoter region or a part thereof including at least one CpG site. The target for analysis may contain only one CpG site; however it is preferable that the target for analysis contains more than one CpG site. The target for analysis may be selected from any one promoter region among the above genes or from promoter regions of more than one gene.

The positions and numbers of CpG sites located in the promoter regions of EFNB2, CCNJ and KCTD 12 genes are already known. Thus the number of methylated CpG site(s) itself in the promoter regions can also be used as the methylation frequency.

The methylation frequency may be "methylation score" obtained by analyzing DNA for methylation status of CpG sites with mass spectrometry such as MassARRAY® as described hereinbelow. MassARRAY® allows calculation of methylation score based on the ratio between the area of a peak derived from a methylated DNA fragment and the area of a peak derived from a non-methylated DNA fragment obtained after measurement of the DNA fragments.

In the embodiments of the present invention, the target for analysis may be any CpG site(s) or certain region(s) including the CpG site(s) in the promoter regions of EFNB2, CCNJ and KCTD12 genes without particular limitation. The positions and numbers of CpG sites located in the promoter regions of the genes are already known. Thus a person skilled in the art may appropriately select the target CpG site or region from the results of routine experiments according to the well known analysis methods described hereinbelow.

Various methods are well-known in the art as to the method for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, the MeDIP method, non-methylated cytosine converting treatment and the like.

The step of amplifying DNA may include the step of carrying out PCR, quantitative PCR, IVT *(in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In embodiments of the present invention, the analyzing step may be the step of concentrating methylated DNA in DNA obtained in the extracting step by the MeDIP method and analyzing methylation status of the concentrated methylated DNA. The methylated DNA concentrated by the MeDIP method may be amplified by e.g. IVT amplification and methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is an agent which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in base sequence (C and U) by the non-methylated cytosine converting treatment using bisulfite. The non-methylated cytosine converting treatment using bisulfite is referred to as bisulfite treatment.

When the bisulfite treatment is carried out, the amount (concentration) of bisulfite added is not specifically limited so long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in base sequence from the original sequence. This process is referred to as bisulfite sequencing method.

Methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in mass (16 Da) due to difference between G and A in the obtained digested fragments is detected on a MALDI-TOF (matrix assisted laser desorption/ionization time-of-flight) mass spectrometer to analyze methylation status of DNA. This method is referred to as MassARRAY® analysis.

It is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY® can be identified as to the portions of the base sequences in the template DNA from which the peaks are originated. For example, when one CpG site is methylated in a DNA fragment, a peak obtained in MassARRAY® shifts to the side with an increased mass for 16 Da. When a DNA fragment containing more than one CpG site is analyzed, for example, the DNA fragment having two methylated CpG sites shows a shift of 32 Da and the DNA fragment having three methylated CpG sites shows a shift of 48 Da.

In mass spectrometry such as MassARRAY®, the methylation score of the analyzed DNA fragment can be calculated. For example when a DNA fragment having a certain sequence results in the ratio between the area of the peaks of non-methylated DNA fragments and the area of the peaks of methylated DNA fragments obtained in a resulting chart from the analysis of 1:3, the methylation score of the DNA fragment is 0.75 (= 3/(1+3)). The methylation score is theoretically 1 for a DNA fragment in which all CpG site(s) is methylated and 0 for a DNA fragment without any methylated CpG site.

Methylation status of CpG sites can be analyzed by a methylation specific PCR (MSP) method. The MSP method is a method in which methylation status of CpG sites (presence or absence of methylation) is analyzed by amplifying DNA after bisulfite treatment by PCR using a primer set described hereinafter and determining presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of EFNB2, CCNJ or KCTD 12 gene on a substrate. The microarray can be prepared according to well-known methods in the art.

In the analysis using a microarray, DNA extracted from a biological sample is preferably labeled with a labeling substance well-known in the art. Thus, the present method preferably further comprises the step of labeling the extracted DNA. The labeling step is advantageously carried out after the DNA amplifying step because all DNA in the biological sample may be labeled. The labeling substance may include fluorescence substances, haptens such as biotin, radioactive substances and the like. The fluorescence substances may include Cy3, Cy5, FITC, Alexa Fluor™ and the like. Labeling of DNA facilitates measurement of a signal from a probe on the microarray. A method for labeling DNA with the labeling substance is well-known in the art.

The above signal may be any suitable signal depending on the type of microarrays. For example, the signal may be an electric signal generated when a DNA fragment hybridizes to a probe on the microarray, or a fluorescence or luminescence signal generated from a labeling substance when DNA to be analyzed is labeled as described above. Detection of a signal can be carried out by using a scanner comprised in a conventional microarray analyzer. The scanner may be, for example, GeneChip® Scanner3000 7G (Affymetrix), Illumina® BeadArray Reader (Illumina) and the like.

In the present method, information on hepatocellular carcinoma in the subject is obtained based on the analysis result obtained in the analyzing step. The information on hepatocellular carcinoma is not particularly limited as far as it may be an index on diagnosis of hepatocellular carcinoma and is preferably information indicative of occurrence or status of hepatocellular carcinoma or both thereof in a subject. The information may include, for example, presence or absence of a cancer cell derived from hepatocellular carcinoma in a biological sample collected from a subject, a possibility of occurrence of hepatocellular carcinoma in a subject, or a risk for future occurrence of hepatocellular carcinoma in a subject. The information on hepatocellular carcinoma in a subject who has already been affected by hepatocellular carcinoma may include prognosis of the subject, a degree of progression (stage) and the like. The information on hepatocellular carcinoma obtained based on the analysis result obtained in the analyzing step does not substantially include information on a type of cancer different from hepatocellular carcinoma because the promoter regions of the above genes which are used as markers in the present method are specifically methylated in cancer cells derived from hepatocellular carcinoma.

In the embodiments of the present invention, the analyzing step which provides the analysis result indicating presence of a methylated CpG site may provide information indicating occurrence of hepatocellular carcinoma or indicating that the status of hepatocellular carcinoma is poor (or aggravated).

In another embodiment of the present invention, the above information can be obtained when the methylation frequency obtained in the analyzing step is at or higher than a certain threshold.

More specifically, the information obtained may be indicative of presence of a cancer cell derived from hepatocellular carcinoma in a biological sample. The information obtained may alternatively indicate that a subject has a high risk for being affected by hepatocellular carcinoma or that a subject has already been affected by hepatocellular carcinoma. For a subject who has already been affected by hepatocellular carcinoma, information obtained may indicate that prognosis of the subject is poor (or aggravated) or that the cancer is in a progressed stage.

On the contrary, when the result from the analyzing step shows absence of methylated CpG site, information suggesting no occurrence of hepatocellular carcinoma or information indicating that hepatocellular carcinoma is in a preferable status can be obtained. Alternatively the above information can be obtained when the methylation frequency obtained in the analyzing step is lower than a certain threshold. More specifically, the information obtained may be indicative of absence of a cancer cell derived from hepatocellular carcinoma in a biological sample. The information obtained may alternatively indicate that a subject has a low risk for being affected by hepatocellular carcinoma or that a subject has not been affected by hepatocellular carcinoma. For a subject who has already been affected by hepatocellular carcinoma, information obtained may be indicative of a preferable prognosis of the subject or indicate that the cancer is in a relatively early stage.

The threshold is not particularly limited and may be empirically established based on accumulated data on various biological samples. The threshold may alternatively be established as follows. First, methylation frequency is analyzed for DNA extracted respectively from a biological sample which is confirmed to be devoid of cancer cells derived from hepatocellular carcinoma (normal liver tissue or normal hepatocyte) and a biological sample containing a cancer cell derived from hepatocellular carcinoma. Next, based on the obtained analysis results, a threshold is established within a range that is higher than the methylation frequency of the biological sample devoid of cancer cells and lower than that of the biological sample containing the cancer cell. Preferably, the threshold is established as a value which can highly accurately differentiate between the biological sample devoid of cancer cells and the biological sample containing the cancer cell.

The scope of the present invention also encompasses a marker for obtaining information on hepatocellular carcinoma by methylation analysis (also merely referred to as "marker"). The marker of the present invention is at least one CpG site selected from CpG sites located in promoter regions of EFNB2, CCNJ and KCTD12 genes.

In the embodiments of the present invention, methylation status of the marker in a DNA sample prepared from a biological sample collected from a subject may be analyzed and information on hepatocellular carcinoma in the subject can be obtained based on the analysis result. The analysis of methylation status and obtainment of information on hepatocellular carcinoma are the same as those previously described herein.

The scope of the present invention encompasses a kit for obtaining information on hepatocellular carcinoma (also merely referred to as "kit"). The kit of the present invention comprises a primer set for analysis of methylation status of at least one CpG site selected from CpG sites located in promoter regions of EFNB2, CCNJ and KCTD 12 genes.

In the embodiments of the present invention, the primer set in the kit may be a primer set for analysis of methylation status of CpG sites according to mass spectrometry such as MassARRAY® or an analysis method involving PCR amplification such as the MSP method, the bisulfite sequencing method, among which the primer set for mass spectrometry such as MassARRAY® or for the MSP is preferred. The base sequences of the primers in the primer set may be appropriately selected by a person skilled in the art based on the base sequences in the promoter regions. Examples of the primer set include a primer set of primers having base sequences SEQ ID NOs: 4 and 5; a primer set of primers having base sequences SEQ ID NOs: 8 and 9; a primer set of primers having base sequences SEQ ID NOs: 10 and 11; a primer set of primers having base sequences SEQ ID NOs: 12 and 13; and a primer set of primers having base sequences SEQ ID NOs: 20 and 21.

The present invention also encompasses a system suitable for provision of information on hepatocellular carcinoma in a subject. For example, the system may be as follows.

A system suitable for provision of information on hepatocellular carcinoma in a subject comprising a computer containing a processor and a memory controlled by the processor, wherein the memory comprises a computer program for enabling the computer to carry out the steps of:

obtaining an analysis result on methylation status of a CpG site located in a promoter region of at least one gene selected from EFNB2, CCNJ and KCTD12 in a DNA sample derived from the subject; and
providing information on hepatocellular carcinoma in the subject based on the resulting analysis result.

The present invention also encompasses a computer program product for enabling a computer to carry out provision of information on hepatocellular carcinoma in a subject. For example, the computer program product may be as follows.

A computer program product for enabling a computer to carry out provision of information on hepatocellular carcinoma in a subject comprising a computer readable medium, wherein the medium comprises a computer program for enabling the computer to carry out the steps of:

obtaining an analysis result on methylation status of a CpG site located in a promoter region of at least one gene selected from EFNB2, CCNJ and KCTD12 in a DNA sample derived from the subject; and
providing information on hepatocellular carcinoma in the subject based on the resulting analysis result.

An embodiment of a suitable device for carrying out the present method is illustrated hereinafter by referring to figures. However, the present invention is not limited only to this embodiment. Fig. 11 is a schematic view of an example of a determination device for providing information on hepatocellular carcinoma in a subject. The determination device 1 shown in Fig. 11 comprises a measurement device 2 and a computer system 3 connected to the measurement device 2.

In the present embodiment, the measurement device 2 is a MALDI-TOF mass spectrometer. The measurement device 2 obtains mass spectrometric information such as the time of flight or the mass-to-charge ratio (m/z ratio) of a substance to be analyzed. The measurement device 2 onto which a measurement sample prepared from the DNA sample derived from a subject is mounted obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2 onto which a gel obtained by electrophoresis of a reaction solution after nucleic acid amplification by the MSP method is mounted detects amplification products. The measurement device 2 then obtains the band intensity data of the amplification products and sends the obtained data to the computer system 3.

The computer system 3 comprises a computer main body 3a, an input device 3b and a display 3c which displays specimen information, determination results and the like. The computer system 3 receives the mass spectrometric information from the measurement device 2. The processor in the computer system 3 executes, based on the mass spectrometric information, a program for providing information on hepatocellular carcinoma in a subject.

Fig. 12 is a block diagram showing the functionality configuration of the determination device of Fig. 11. As shown in Fig. 12, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a threshold necessary for determination and a formula for calculating the methylation score. The calculating unit 303 calculates the methylation score from information obtained at the receiving unit 301 according to the formula stored in the memory unit 302. The determining unit 304 determines whether or not the methylation score calculated at the calculating unit 303 is lower than the threshold stored at the memory unit 302. The output unit 305 outputs the determination result from the determining unit 304 as information on hepatocellular carcinoma in the subject (e.g., presence or absence of a cancer cell derived from hepatocellular carcinoma in the biological sample collected from the subject).

Fig. 13 is a block diagram showing the hardware configuration of the determination device in Fig. 11. As shown in Fig. 13, the computer main body 3a comprises a CPU (Central Processing Unit) 30, ROM (Read Only Memory) 121, ROM 32, a hard disk 33, an input/output interface 34, a read-out device 35, a communication interface 36 and an image output interface 37. The CPU 30, ROM 31, RAM (Random Access Memory) 32, the hard disk 33, the input/output interface 34, the read-out device 35, the communication interface 36 and the image output interface 37 are connected via a bus 38 so as to be able to communicate with each other.

The CPU 30 can execute a computer program stored in ROM 31 and a computer program loaded with ROM 32. When the CPU 30 executes the application program, the functional blocks described above may be executed. Accordingly the computer system serves as a terminal that is a determination device for providing information on hepatocellular carcinoma in a subject.

ROM 31 is made up with mask ROM, PROM, EPROM, EEPROM or the like. ROM 31 stores the computer program executed by the CPU 30 and data used for the execution.

ROM 32 is made up with SRAM, DRAM or the like. ROM 32 is used for readout of the computer programs stored in ROM 31 and the hard disk 33. ROM 32 is also utilized as a work area when the CPU 30 executes these computer programs.

An operating system to be executed by the CPU 30, computer programs such as application programs (the computer program for providing information on hepatocellular carcinoma in a subject) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analog interface formed by a D/A converter, an A/D converter or the like. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

The process operations carried out by the determination device 1 for providing information on hepatocellular carcinoma in a subject are illustrated hereinafter. Fig. 14 is a flow chart for providing information on hepatocellular carcinoma using the determination device of Fig. 11. An example is herein illustrated in which mass spectrometric information of a nucleic acid in a measurement sample prepared from a DNA sample derived from a subject is used for calculation of a peak area from which a methylation score is calculated and the methylation score is determined as to whether or not it is lower than a threshold. However, the present invention is not limited only to this embodiment.

In the step S1-1, the receiving unit 301 in the determination device 1 receives from the measurement device 2 mass spectrometric information. In the next step S1-2, the calculating unit 303 calculates from the mass spectrometric information received at the receiving unit 301 a peak area which is sent to the memory unit 302. In the step S1-3, the calculating unit 303 calculates the methylation score based on the peak area stored in the memory unit 302 according to the formula stored in the memory unit 302.

In the step S1-4, the determining unit 304 determines whether or not the methylation score calculated at the calculating unit 303 is lower than the threshold stored in the memory unit 302. When the methylation score is lower than the threshold, the determining unit 304 in the step S1-5 sends a determination result indicating that the biological sample collected from the subject does not contain a cancer cell derived from hepatocellular carcinoma to the output unit 305. When the methylation score is not lower than the threshold (i.e., the methylation score is at or higher than the threshold), the determining unit 304 sends a determination result indicating that the biological sample collected from the subject contains a cancer cell derived from hepatocellular carcinoma to the output unit 305.

In the step S1-7, the output unit 305 outputs the determination result as information on hepatocellular carcinoma in the subject, so that the display 3c displays the result and/or the printer prints out the result. Accordingly, the determination device can provide, to a physician or the like, information assisting the physician or the like to judge whether or not the subject has hepatocellular carcinoma.

The present invention is specifically described hereinafter by way of Examples which do not limit the present invention.

Examples

Reference Example 1: Identification of novel markers from genomic DNA of hepatocellular carcinoma tissues

(1) Biological samples

In the present Reference Example, the biological samples used were clinical specimens obtained from patients by hepatectomy. The clinical specimens contained cancerous tissues of hepatocellular carcinoma (100 specimens), normal liver tissues (3 specimens) and chronic hepatitis/cirrhosis tissues (39 specimens). The tissues were immediately frozen with liquid nitrogen after collection and stored at -80°C prior to use.

(2) DNA extraction and bisulfite treatment

Genomic DNA was extracted with the QIAamp DNA Mini Kit (QIAGEN) from the above tissues. The obtained genomic DNA (500 ng) was subjected to bisulfite treatment with the EZ DNA Methylation Kit (Zymo Research) and the genomic DNA after treatment was eluted in sterilized distilled water (10 $\mu$l). The genomic DNA contained in the obtained DNA solution (4 $\mu$l) was fragmented with Bioruptor (COSMO BIO).

(3) Identification of novel markers

The fragmented genomic DNA was subjected to Infinium Methylation Assay using the Infinium HumanMethylation27 BeadChip (Illumina) in order to search novel markers which are specifically methylated in cancerous tissues of hepatocellular carcinoma. The specific procedures carried out followed the manual attached to the chip.

The Infinium HumanMethylation27 BeadChip includes probes for methylated and non-methylated CpG sites for each of 27,578 CpG sites on the human genome. In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in the respective genes was calculated according to the following calculation formula:

$$(\text{mCpG}) = (\text{signal M})/\{(\text{signal M}) + (\text{signal U})\}$$

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the respective genes in various tissues was calculated according to the following formula: Methylation positive rate (%) = (number of methylation positive specimens/total number of specimens) x 100

For example, for cancerous tissue specimens, the methylation positive rate of a gene can be calculated by "(number of methylation positive specimens among cancerous tissue specimens/total number of cancerous tissue specimens) x 100". The gene which showed a statistically significant difference between cancerous tissue specimens and cirrhosis tissue specimens or normal tissue specimens was identified as a marker which is methylated in cancerous tissues.

As a result, promoter regions of EFNB2, CCNJ and KCTD12 genes were identified as markers which are highly methylated in cancerous tissues of hepatocellular carcinoma (see Figs. 1A to 1C). These markers may also be referred to as the present markers hereinbelow.

Reference Example 2: Comparison of methylation data between cancer/tumor tissue specimens derived from several types of cancer/tumor, non-cancerous tissue specimens and normal tissue specimens

(1) Collection of methylation data

In the present Reference Example, methylation data of 9 types of cancer/tumor tissue specimens, 6 types of non-cancerous tissue specimens and 13 types of normal tissue specimens excluding cancerous tissue specimens derived from hepatocellular carcinoma, non-cancerous tissue specimens derived from hepatocellular carcinoma and normal tissue specimens derived from normal liver were compared. The number of specimens for the respective tissues is shown in the following tables.

Table 2

| Cancer/tumor tissue specimens | |
|---|---|
| **Tissue** | **No. of specimens** |
| **Brain tumor (Brain)** | 283 |
| **Breast cancer (Breast)** | 186 |
| **Lung cancer (Lung)** | 59 |
| **Gastric cancer (Gastric)** | 82 |
| **Colon cancer (Colon)** | 236 |
| **Renal cell carcinoma (Kidney)** | 219 |
| **Uterine cancer (Uterus)** | 70 |
| **Ovarian cancer (Ovary)** | 519 |
| **Prostate cancer (Prostate)** | 93 |

Table 3

| Non-cancerous tissue | |
|---|---|
| **Tissue** | **No. of specimens** |
| **Lung** | 59 |
| **Colonic mucosa (Colon)** | 16 |
| **Kidney** | 199 |
| **Uterus** | 1 |
| **Ovary** | 16 |
| **Prostate** | 87 |

Table 4

| Normal tissue | |
|---|---|
| **Tissue** | **No. of specimens** |
| **Normal brain (Brain)** | 2 |
| **Normal oral mucosa (Oral)** | 2 |
| **Normal lung (Lung)** | 2 |
| **Normal colonic mucosa (Colon)** | 2 |
| **Normal liver (Liver)** | 2 |
| **Peripheral blood from healthy subject (Blood)** | 2 |
| **Normal skeletal muscle (Skeletal)** | 2 |
| **Normal testis (Testis)** | 1 |
| **Normal gastric mucosa (Stomach)** | 2 |
| **Normal pancreas (Pancreas)** | 1 |

(continued)

| Normal tissue | |
|---|---|
| **Tissue** | **No. of specimens** |
| **Normal spleen (Spleen)** | 1 |
| **Normal kidney (Kidney)** | 2 |
| Peripheral blood from healthy subject (Blood) * (Blood, Sahia B, et al.) | 93 |

The methylation data for cancerous tissue specimens derived from hepatocellular carcinoma, non-cancerous tissue specimens derived from hepatocellular carcinoma, normal tissue specimens derived from normal liver and 12 types of normal tissue specimens were those measured by Infinium Methylation Assay using the Infinium HumanMethylation27 BeadChip (Illumina) in the same manner as described in Reference Example 1. The methylation data for other specimens were the methylation data of the Infinium HumanMethylation27 BeadChip (Illumina) available from TCGA (The Cancer Genome Atlas: http://tcga-data.nci.nih.gov/tcga/tcgaHome2.jsp) or, for the specimens of lung cancer and prostate cancer and 93 specimens of peripheral blood from healthy subjects, published in the following references. The methylation data in this context are the methylation rate (mCpG) of CpG sites in EFNB2, CCNJ and KCTD 12 obtained as described in section (3) in Reference Example 1.

- Lung cancer: Selamat SA et al., Genome-scale analysis of DNA methylation in lung adenocarcinoma and integration with mRNA expression. Genome Res. Jul; 22 (7): 1197-211 (2012).
- Prostate cancer: Kobayashi Y et al., DNA methylation profiling reveals novel biomarkers and important roles for DNA methyltransferases in prostate cancer. Genome Res. Jul 21 (7): 1017-27 (2011).
- Data of 93 specimens of peripheral blood from healthy subjects: Salhia B et al., DNA methylation analysis determines the high frequency of genic hypomethylation and low frequency of hypermethylation events in plasma cell tumors. Cancer Res. Sep 1; 70 (17): 6934-44 (2010).

(2) Comparison of methylation positive rate between cancer/tumor tissue specimens derived from several types of cancer/tumor, non-cancerous tissue specimens and normal tissue specimens

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the present markers in various tissues was calculated according to the above formula. The results are shown in Figs. 2A to 2C. In Fig. 2, "Normal tissue" represents, among the tissues indicated in Table 4, the normal tissues excluding 93 specimens of peripheral blood from healthy subjects (Salhia B et al.) and "Normal blood" represents the 93 specimens of peripheral blood from healthy subjects (Salhia B et al.).

Figs. 2A to 2C show that all of the present markers are not highly methylated in other types of cancer or human normal tissues or human normal blood (peripheral blood from healthy subjects, Salhia B et al.) and thus are specifically and highly methylated in hepatocellular carcinoma.

Comparative Example 1: Comparison of methylation positive rate between cancer/tumor tissue specimens derived from several types of cancer/tumor, non-cancerous tissue specimens and normal tissue specimens

The methylation positive rate was calculated for each of GSTP1, CDKN2A and CDH1 genes (hereinafter referred to as "known markers") which have already been known to be methylated in cancer cells derived from hepatocellular carcinoma in the similar manner as Reference Example 2 in the respective tissues. The results are shown in Figs. 3A to 3C.

According to Figs. 3A and 3B, GSTP1 and CDKN2A showed high positive rates in hepatocellular carcinoma and were also methylated in other types of cancer and other non-cancerous tissues. Therefore, these genes have low specificity towards hepatocellular carcinoma. According to Fig. 3C, CDH1 showed low positive rate in hepatocellular carcinoma and was also methylated in other types of cancer and non-cancerous tissues, resulting in low sensitivity and specificity. Thus, the known markers have high sensitivity while having low specificity as markers of hepatocellular carcinoma, thereby posing issues in terms of performance as diagnostic markers of hepatocellular carcinoma. Namely it was shown that a search for markers is required to be carried out by taking the specificity in other types of cancer and other non-cancerous tissues into account.

Example 1: Comparison of methylation data (MassARRAY) between normal liver, cirrhosis and hepatocellular carcinoma

(1) Biological samples

In the present Example, the biological samples used were clinical specimens obtained from patients different from those used in Reference Example 1 by hepatectomy. The clinical specimens contained normal liver tissues (2 specimens), cirrhosis tissues (5 specimens) and cancerous tissues of hepatocellular carcinoma (6 specimens). The tissues were immediately frozen with liquid nitrogen after collection and stored at -80°C prior to use.

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO). In order to generate a calibration curve for mass spectrometry, the control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA). By mixing the 0% methylated DNA solution and the 100% methylated DNA solution at certain proportions, solutions of 25%, 50% and 75% methylated DNA were obtained.

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(iii) Amplification by PCR and IVT

Methylated cytosine and uracil in the DNA fragments after bisulfite treatment were converted respectively to guanine and adenine by PCR and IVT amplification.

It is verified by MassARRAY® analysis using control samples as described hereinbelow that the primer set used for PCR can amplify both methylated DNA and non-methylated DNA without bias. The sequences of the primer set for the present marker are shown in Table 5. The base sequence (sequence of the negative strand) which can be analyzed with the primer set is shown in SEQ ID NO: 14.

Table 5

| Marker | Base sequence of primer | SEQ ID NO: |
|---|---|---|
| *EFNB2* | **Forward :** GGTTTTGATTAGAGTTGTAA | 4 |
| | **Reverse :** CAATTTCAATCAATAAAACC | 5 |

The following tag sequence and T7 promoter sequence were respectively added to the 5'-terminals of the forward primer and reverse primer in the above primer set for IVT reaction.

- Tag sequence: AGGAAGAGAG (SEQ ID NO: 6)
- T7 promoter sequence: CAGTAATACGACTCACTATAGGGAGAAGGCT (SEQ ID NO: 7)

PCR reaction solution was prepared by mixing the following reagents:

| | |
|---|---|
| 10 x Hot Star buffer (QIAGEN) | 0.5 μL |
| 25 mM dNTP mix | 0.04 μL |
| Hot Star Taq (5 U/μL) (QIAGEN) | 0.04 μL |
| Primer mix | 2.0 μL |

(continued)

| | |
|---|---|
| DNA solution | 1.0 μL |
| Water | 1.42 uL |
| Total | 5 μL |

PCR reaction was carried out on the reaction solution under the following conditions:

1 cycle of 94°C for 15 minutes;
45 cycles of 94°C for 20 seconds, 52°C for 30 seconds and 72°C for 1 minute; and 72°C for 3 minutes.

The obtained PCR products were dephosphorylated with SAP (Shrimp Alkaline Phosphatase) in the MassCLEAVE™ Reagent kit (SEQUENOM). The following reaction solution prepared with the kit was then added.

| | |
|---|---|
| 5 x T7 R&DNA polymerase buffer | 0.89 μL |
| T Cleavage mix | 0.24 μL |
| 100 mM DTT | 0.22 μL |
| T7 R&DNA polymerase | 0.44 μL |
| RNase A | 0.06 μL |
| RNase-free water | 3.15 μL |
| Total | 5 μL |

The obtained mixture was incubated at 37°C for 3 hours to effect IVT reaction and uracil or thymine specific cleavage. The obtained reaction product was purified with Clean Resin (SEQUENOM) to obtain a sample for mass spectrometry. Samples derived from genomic DNA extracted from the above clinical specimens were designated as measurement samples and samples derived from control genomic DNA were designated as control samples, both of which were used for mass spectrometry described hereinbelow.

(3) Analysis of methylation status by mass spectrometry using MassARRAY®

(i) Preparation of calibration curve

Two mass spectrometric analyses were carried out independently on the control samples obtained as above. On the basis of the obtained analysis results calibration curves were prepared for the primer set and the correlation coefficient was calculated. Accordingly it was verified that the primer set used could amplify both methylated DNA and non-methylated DNA without bias.

(ii) Analysis of measurement samples

The measurement samples obtained as above were subjected to mass spectrometry to obtain peaks of DNA fragments contained in the measurement samples. The obtained peaks were then allocated to the portions of the base sequence of EFNB2. The methylation score was calculated on the basis of the ratio between the area of the peaks of the fragments containing a methylated CpG site and the area of the peaks of the fragments without the methylated CpG site, both fragments having the same base sequence. The obtained results are shown in Fig. 4.

According to Fig. 4, it was found that EFNB2 showed low methylation score in normal liver tissues and cirrhosis tissues while high methylation score in hepatocellular carcinoma. It was therefore demonstrated that the methylation score of the present marker correlates with hepatocellular carcinoma, similar to the results of the Infinium method in Reference Example 1. In addition, according to Fig. 4, it was found that analysis of methylation frequency of the present marker allows establishment of the threshold that can distinguish specimens of hepatocellular carcinoma and specimens of normal liver tissues or cirrhosis tissues.

Example 2: Comparison of methylation data (MSP) between normal liver tissues, cirrhosis tissues and hepatocellular carcinoma tissues (1) Biological samples

In the present Example, the biological samples used were normal liver tissues (2 specimens), cirrhosis tissues (2 specimens) and cancerous tissues of hepatocellular carcinoma (6 specimens) as in Example 1.

(2) Preparation of measurement samples and control samples

(i) Extraction of genomic DNA

Genomic DNA was extracted from the above tissues with the QIAamp DNA Mini Kit (QIAGEN). The genomic DNA contained in the obtained DNA solution was fragmented with Bioruptor (COSMO BIO).

The control genomic DNA used was genomic DNA of human peripheral blood lymphocytes. The genomic DNA from human peripheral blood lymphocytes was amplified with the GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the solution of the non-methylated DNA fragments was subjected to reaction with SssI methylase (New England Biolab) to methylate all cytosines in CG sequences and obtain a solution of methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) Methylation specific PCR (MSP)

MSP was carried out on the measurement samples and control samples (DNA after bisulfite treatment) obtained in the above section (2). The composition of PCR reagents, primer sets and reaction conditions for PCR in MSP are shown below.

<PCR reagent>

| | |
|---|---|
| DDW (sterilized water) | 16.8 μL |
| 10 x PCR buffer with $MgCl_2$ (Roche) | 2.5 μL |
| 2 mM dNTP mix | 2.5 μL |
| 10 μM sense primer | 1.0 μL |
| 10 μM antisense primer | 1.0 μL |
| Faststart Taq polymerase (Roche) | 0.2 μL |
| Measurement sample or control sample | 1.0 uL |
| Total | 25 μL |

<Primer set>

The primer sets used for MSP are shown in Table 6. These primer sets allow generation of amplification products when DNA in the amplified regions is methylated. A primer set for accuracy control was also used which allows judgment on whether or not the bisulfite treatment had been appropriately carried out (see Table 7). The base sequences which can be analyzed with the primer sets shown in Table 6 in the promoter regions in EFNB2, CCNJ and KCTD12 genes are shown in SEQ ID NOs: 17 to 19, respectively (the base sequences SEQ ID NOs: 17 and 18 correspond to the sequences of negative strands and the base sequence SEQ ID NO: 19 corresponds to the sequence of the positive strand).

Table 6

| Gene symbol | Primer | Base sequence of primer | SEQ ID NO: | (Size of PCR product (bp) | Annealing temp. (°C) | Cycles |
|---|---|---|---|---|---|---|
| *EFNB2* | EFNB2_MF | AATTTTTGGTTATTTTGGGAAGC | 8 | 182 | 58 | 36 |
| | EFNB2_MR | CTAATCAAAACCTAAACATCGTCG | 9 | | | |
| *CCNJ* | CCNJ_MF | GTTTTAGAAAGGGGAATAAAAGGTC | 10 | 93 | 60 | 36 |
| | CCNJ_MR | CCGACAAACCTAAAATAAACGTA | 11 | | | |
| *KCTD12* | KCTD12_MF | GTCGGGATAGTGGTAGGAAGTC | 12 | 154 | 62 | 32 |
| | KCTD12_MR | GCGCGACTCTAAAAAATAACG | 13 | | | |

Table 7

| Base sequence of primer | | SEQ ID NO: | Size of PCR product (bp) | Annealing temp. (°C) | Cycles |
|---|---|---|---|---|---|
| **Forward** | GGGATATTAAGTGGAGTTATTTTGGTTTTAGTT | 15 | 129 | 60 | 40 |
| **Reverse** | CCCTCCCAACATCCTTCCTAA | 16 | | | |

<PCR reaction conditions>

95°C for 6 minutes;
Y cycles of 95°C for 30 seconds, X°C for 30 seconds and 72°C for 30 seconds; 72°C for 7 minutes; and
keep at 16°C.

In the above reaction conditions, "X" and "Y" respectively represent the annealing temperature and the number of cycles as indicated in Tables 6 and 7.

(4) Analysis of results of methylation specific PCR (MSP)

The amplification product from MSP was verified by 2% agarose gel electrophoresis. The results are shown in Fig. 5. In this figure, "0" and "100" under "control" represent the 0% methylation control sample and the 100% methylation control sample, respectively.

In PCR using the primer set for accuracy control, bands were detected in all lanes as shown in Fig. 5. This shows that bisulfite treatment of the samples was appropriately carried out. In PCR using the present markers, bands derived from methylated CpGs were not detected for any normal liver tissues or cirrhosis tissues. The band appeared in the lane KCTD12_M, normal liver 2 corresponds to primer dimer. In contrast, PCR of hepatocellular carcinoma tissues resulted in detection of bands in 4 samples among 6 samples, 4 samples among 6 samples and 3 samples among 6 samples for the markers of EFNB2, CCNJ and KCTD12, respectively. Accordingly it is indicated that in methylation analysis of the present markers by MSP method, methylation of the present markers and hepatocellular carcinoma are correlated, similar to the result of the Infinium method from Reference Example 1. Namely it is suggested that EFNB2, CCNJ and KCTD12 are markers with high specificity such that they tend to be highly methylated in hepatocellular carcinoma but methylation thereof is not detected in other normal tissues.

Reference Example 3: Analysis of CpG sites in promoter region of EFNB2 gene

In the present Reference Example, CpG sites in the promoter region of EFNB2 gene were further analyzed by utilizing the methylation data on the Infinium HumanMethylation450 BeadChip (Illumina).

(1) Biological samples

In the present Reference Example, the biological samples used were clinical specimens obtained from patients by hepatectomy. The clinical specimens contained cancerous tissues of hepatocellular carcinoma (99 specimens), non-cancerous tissues of hepatocellular carcinoma (19 specimens) and normal liver tissues (2 specimens). The tissues were immediately frozen with liquid nitrogen after collection and stored at -80°C prior to use.

(2) DNA extraction and bisulfite treatment

Genomic DNA was extracted with the QIAamp DNA Mini Kit (QIAGEN) from the above tissues. The obtained genomic DNA (500 ng) was subjected to bisulfite treatment with the EZ DNA Methylation Kit (Zymo Research) and the genomic DNA after treatment was eluted in sterilized distilled water (10 μl). The genomic DNA contained in the obtained DNA solution (4 μl) was fragmented with Bioruptor (COSMO BIO).

(3) Calculation of methylation positive rate of CpG site of EFNB2

The fragmented genomic DNA was subjected to Infinium Methylation Assay using the Infinium HumanMethylation450 BeadChip (Illumina). The specific procedures carried out followed the manual attached to the chip. The Infinium HumanMethylation450 BeadChip includes probes that allow quantification of methylation status of 482,421 CpG sites on human genome. The Infinium HumanMethylation450 BeadChip is designed to allow quantification of signal intensity of methylated DNA fragments and non-methylated DNA fragments for respective CpG sites by means of hybridization to probes and single base extension reaction. The 265,824 probes corresponding to about 55% of all probes target the regions within 5 kb from the transcription initiation sites of genes (10.1 probes per gene in average). In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in EFNB2 was calculated according to the following calculation formula:

$$(\text{mCpG}) = (\text{signal M})/\{(\text{signal M}) + (\text{signal U})\}$$

The threshold was set as "0.4" for the methylation rate (mCpG) of CpG sites of EFNB2, and a specimen having a methylation rate at or higher than the threshold was designated as "methylation positive specimen". Based on the number of methylation positive specimens, methylation positive rate (%) for EFNB2 gene in various tissues was calculated according to the following formula:

$$\text{Methylation positive rate (\%)} = (\text{number of methylation positive specimens/total number of specimens}) \times 100$$

The methylation positive rate of CpG sites of EFNB2 in each tissue is shown in Fig. 6. According to Fig. 6, the CpG sites of EFNB2 showed high methylation positive rate only in cancerous tissues of hepatocellular carcinoma. This indicates that in the promoter region of EFNB2 gene, CpG sites which are not analyzed on the Infinium HumanMethylation27 BeadChip are highly methylated in cancerous tissues of hepatocellular carcinoma.

Reference Example 4: Comparison of methylation data between cancer/tumor tissue specimens derived from several types of cancer/tumor, non-cancerous tissue specimens and normal tissue specimens

(1) Collection of methylation data

In the present Reference Example, methylation data of 7 types of cancer/tumor tissue specimens, 7 types of non-cancerous tissue specimens and 19 types of normal tissue specimens were compared. The number of specimens for the respective tissues is shown in the following tables.

Table 8

| Cancer/tumor tissue specimens | |
|---|---|
| Tissue | No. of specimens |
| Brain tumor (Brain) | 114 |
| Breast cancer (Breast) | 548 |
| Squamous cell lung cancer (Lung) | 150 |
| Liver cancer (Liver) | 99 |
| Colon cancer (Colon) | 324 |
| Uterine body cancer (Uterus) | 334 |
| Renal clear cell carcinoma (Kidney) | 282 |

Table 9

| Non-cancerous tissue | |
|---|---|
| Tissue | No. of specimen |
| Brain tumor (Brain) | 2 |
| Breast cancer (Breast) | 98 |
| Squamous cell lung cancer (Lung) | 43 |
| Liver cancer (Liver) | 19 |
| Colon cancer (Colon) | 40 |
| Uterine body cancer (Uterus) | 36 |
| Renal clear cell carcinoma (Kidney) | 164 |

Table 10

| Tissue | RCAST | Literature 1 | Literature 2 | Total |
|---|---|---|---|---|
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colonic mucosa (Colon) | 2 | 0 | 0 | 2 |
| Normal liver (Liver) | 2 | 0 | 0 | 2 |
| Peripheral blood from healthy subjects (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal gastric mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |
| Normal adrenal gland (Adrenal gland) | 0 | 2 | 0 | 2 |
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose tissue (adipose tissue) | 0 | 2 | 0 | 2 |
| Normal heart (Heart) | 0 | 1 | 0 | 1 |
| Various normal blood cell components (WB, PBMC, Gran, CD4+, CD8+, CD14+, CD19+, CD56+, Neu, Eos) | 0 | 0 | 60 | 60 |

In Table 10, the methylation data for the specimens indicated in the column "RCAST" were obtained by the present inventors according to Infinium Methylation Assay using the Infinium HumanMethylation450 BeadChip (Illumina). The methylation data for the specimens indicated in the columns "Literature 1" and "Literature 2" were the methylation data published in the following literatures obtained with the Infinium HumanMethylation450 BeadChip. The methylation data in this context are the methylation rate (mCpG) of CpG sites in EFNB2 obtained as described in section (3) in Reference Example 3. The average of the positive rate was plotted for the 18 types of normal tissues excluding the normal blood cell components. For normal blood cell components, the average of 60 specimens of the respective blood cell components from healthy subjects (6 subjects x 10) was plotted.

- Literature 1: Nazor KL et al., Recurrent variations in DNA methylation in human pluripotent stem cells and their differentiated derivatives. Cell Stem Cell 2012; 10 (5): 620-634
- Literature 2: Reinius LE et al., Differential DNA Methylation in Purified Human Blood Cells: Implications for Cell Lineage and Studies on Disease Susceptibility, PLoS One, 7(7) e41361

(2) Comparison of methylation positive rate between cancer/tumor tissue specimens derived from several types of cancer/tumor, non-cancerous tissue specimens and normal tissue specimens

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for EFNB2 in various tissues was calculated according to the above formula. The results are shown in Fig. 7. In Fig. 7, "Normal tissues" represent, among the tissues indicated in Table 10, the normal tissues excluding 60 specimens of various normal blood cell components and "Normal blood cells" represent the 60 specimens of various normal blood cell components.

Fig. 7 shows that CpG sites of EFNB2 are rarely methylated in other types of cancer or human normal tissues or human normal blood cells and thus are specifically and highly methylated in hepatocellular carcinoma.

Comparative Example 2: Comparison of methylation positive rate between cancer/tumor tissue specimens derived from several types of cancer/tumor, non-cancerous tissue specimens and normal tissue specimens

The methylation positive rate was calculated for GSTP1, CDKN2A and CDH1 genes which are known markers in the similar manner as Reference Example 4 in the respective tissues using the Infinium HumanMethylation450 BeadChip (Illumina). The results are shown in Figs. 8A to 8C.

According to Figs. 8A to 8C, methylation of CDH1, CDKN2A and GSTP1 were detected in other types of cancer than hepatocellular carcinoma and other non-cancerous tissues, and thus the genes have low specificity towards hepatocellular carcinoma. Thus known markers have issues in terms of performance as diagnostic markers of hepatocellular carcinoma.

Example 3: Methylation analysis of promoter region of EFNB2 gene by MSP

In the present Example, methylation analysis was carried out by MSP for a part of the promoter region in EFNB2 gene other than the one analyzed in Example 2.

(1) Biological samples

In the present Example, the biological samples used were cancerous tissues collected from hepatocellular carcinoma patients (6 specimens). The control samples used were normal liver tissues (2 specimens) and non-cancerous tissues collected from hepatocellular carcinoma patients (5 specimens).

(2) Preparation of measurement samples and control samples

(i) Extraction of genomic DNA

Genomic DNA was extracted from the above tissues with the QIAamp DNA Mini Kit (QIAGEN). Specifically, according to the protocol attached to the kit, each tissue sample was completely lysed in a lysis buffer containing Proteinase K and RNase A and the resulting solution was subjected to absorption on a column. The column was then washed with a washing buffer followed by elution of genomic DNA with 200 μl of sterilized distilled water to obtain purified genomic DNA. The obtained genomic DNA was fragmented with Bioruptor (COSMO BIO).

The control genomic DNA used was genomic DNA of human peripheral blood lymphocytes. In the same manner as in Example 2, the genomic DNA from human peripheral blood lymphocytes was used to prepare non-methylated DNA fragments (0% methylated DNA) and methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) MSP

MSP was carried out on the measurement samples and control samples (DNA after bisulfite treatment) obtained in the above section (2). The composition of PCR reagents used for MSP is the same as in Example 2. The primer sets and reaction conditions for PCR are shown below.

<Primer set>

The primer sets used for MSP are shown in Table 11. The primer set for EFNB2 allows generation of amplification products when DNA in the amplified regions is methylated. A primer set for accuracy control was also used which allows judgment on whether or not the bisulfite treatment had been appropriately carried out. The base sequence (sequence of the negative strand) which can be analyzed with the primer set shown in Table 11 in the promoter region of EFNB2 gene is shown in SEQ ID NO: 24.

Table 11

| Primer | | Base sequence of primer | SEQ ID NO: | Annealing temp. (°C) | Cycles |
|---|---|---|---|---|---|
| *EFNB2* | Sense | AGCGAGCGTATTTGGTTC | 20 | 60 | 34 |
| | Antisense | ATCCTCCGAAACAAACTAACG | 21 | | |
| Primers for accuracy control | Sense | GGGATATTAAGTGGAGTTATTTTGGTTTTAGTT | 22 | 60 | 36 |
| | Antisense | CCCTCCCAACATCCTTCCTAA | 23 | | |

<PCR reaction conditions>

95°C for 6 minutes;
Y cycles of 95°C for 30 seconds, X°C for 30 seconds and 72°C for 30 seconds; 72°C for 7 minutes; and keep at 16°C.

In the above reaction conditions, "X" and "Y" respectively represent the annealing temperature and the number of cycles as indicated in Table 11.

(4) Analysis of results of MSP

The amplification product from MSP was verified by 2% agarose gel electrophoresis. The results are shown in Fig. 9. In this figure, "0" and "100" under "control" represent the 0% methylation control sample and the 100% methylation control sample, respectively.

According to Fig. 9, bands were detected in all samples of hepatocellular carcinoma tissues while bands derived from methylated CpGs were not detected in normal liver tissues or non-cancerous tissues. Accordingly, it was found that CpG sites in the promoter region of the present marker, EFNB2, are highly methylated in hepatocellular carcinoma tissues as detected by methylation analysis by MSP, and thus the marker has high specificity and sensitivity. It is believed that the present marker is a novel marker sufficiently comparable to known hepatocellular carcinoma markers.

Example 4: Methylation analysis by MSP using blood samples

In the present Example, methylation analysis was carried out by MSP for CpG sites in promoter regions of EFNB2 and CCNJ using peripheral blood as a biological sample.

(1) Biological samples

In the present Example, the biological samples used were peripheral blood collected from patients with hepatocellular carcinoma (6 specimens). The control samples used were peripheral blood from healthy subjects (3 specimens).

(2) Preparation of measurement samples and control samples

(i) Extraction of genomic DNA

Plasma was obtained from each of the above peripheral blood (2 ml) according to the standard method. Genomic DNA was extracted from the resulting plasma with the QIAamp Circulating Nucleic Acid Kit (QIAGEN). The obtained genomic DNA contained in the obtained DNA solution was fragmented with Bioruptor (COSMO BIO).

The control genomic DNA used was genomic DNA of human peripheral blood lymphocytes. In the same manner as in Example 2, the genomic DNA from human peripheral blood lymphocytes was used to prepare non-methylated DNA fragments (0% methylated DNA) and methylated DNA fragments (100% methylated DNA).

(ii) Bisulfite treatment

The respective DNA fragments (500 ng) obtained as above were subjected to bisulfite treatment with the EZ DNA Methylation Kit (Zymo Research) and the treated genomic DNA was eluted in sterilized distilled water (80 μl).

(3) MSP

MSP was carried out on the measurement samples and control samples (DNA after bisulfite treatment) obtained in the above section (2). The composition of PCR reagents used for MSP is the same as in Example 2.

The primer sets used were the primer sets for EFNB2 and CCNJ indicated in Tables 6 and 11. The reaction conditions for PCR are shown below.

<PCR reaction conditions>

95°C for 6 minutes;
50 cycles of 95°C for 30 seconds, 60°C for 30 seconds and 72°C for 30 seconds;
72°C for 7 minutes; and
keep at 16°C.

(4) Analysis of results of MSP

The amplification product from MSP was verified by 2% agarose gel electrophoresis. The band intensities of amplification products were represented in graphs (see Figs. 10A to 10D). The bars in Figs. 10A to 10C indicated under peripheral blood of healthy subjects correspond to the background, indicating absence of detection of any band. According to Figs. 10A to 10C, it is found that PCR for EFNB2 and CCNJ allowed detection of no band in peripheral blood from healthy subjects and detection of bands in at least 3 samples among 6 samples of peripheral blood from hepatocellular carcinoma patients. Fig. 10D indicates that bands were detected in all samples by PCR with the primer set for accuracy control. This indicates that the bisulfite treatment of the samples had been appropriately carried out. Accordingly it is found that the present markers tend to be highly methylated in hepatocellular carcinoma patients and methylation thereof is not detected in healthy subjects even when the biological sample used is peripheral blood. Therefore it is suggested that the present markers are highly specific for hepatocellular carcinoma and are useful for determining presence or absence of cancer cells derived from hepatocellular carcinoma in blood samples. Reference Signs List

1 Determination device
2 Measurement device
3 Computer system
3a Computer main body
3b Input device
3c Display

## Claims

1. A method for obtaining information on hepatocellular carcinoma comprising the steps of:

   preparing a DNA sample from a biological sample collected from a subject;
   analyzing methylation status of a CpG site in a promoter region of at least one gene selected from EFNB2, CCNJ and KCTD 12 in the DNA sample obtained from the preparation step; and
   obtaining information on hepatocellular carcinoma in the subject based on an analysis result obtained from the analyzing step.

2. The method according to claim 1, wherein the analyzing step is the step of analyzing presence or absence of methylation of at least one CpG site.

3. The method according to claim 2, wherein
   the information on hepatocellular carcinoma in the subject is presence or absence of a cancer cell derived from hepatocellular carcinoma in the biological sample collected from the subject, and
   the step of obtaining information is the step of obtaining information indicating that the biological sample contains a cancer cell derived from hepatocellular carcinoma when the analysis result shows that there is a methylated CpG site.

4. The method according to claim 1, wherein the analyzing step is the step of analyzing methylation frequency.

5. The method according to claim 4, wherein
   the information on hepatocellular carcinoma in the subject is presence or absence of a cancer cell derived from hepatocellular carcinoma in the biological sample collected from the subject, and
   the step of obtaining information is the step of obtaining information indicating that the biological sample contains a cancer cell derived from hepatocellular carcinoma when the methylation frequency obtained from the analyzing step is higher than a predetermined threshold.

6. A marker for obtaining information on hepatocellular carcinoma by methylation analysis, which is at least one CpG site selected from CpG sites located in promoter regions of EFNB2, CCNJ and KCTD 12 genes.

7. A kit for obtaining information on hepatocellular carcinoma, comprising a primer set for analysis of methylation of at least one CpG site selected from CpG sites located in promoter regions of EFNB2, CCNJ and KCTD12 genes.

8. The kit according to claim 7, wherein the primer set is a primer set for analyzing methylation status of the CpG site by at least one method selected from mass spectrometry and methylation specific PCR method.

9. The kit according to claim 8, wherein the primer set is at least one selected from:

   a primer set of primers respectively having base sequences SEQ ID NOs: 4 and 5;
   a primer set of primers respectively having base sequences SEQ ID NOs: 8 and 9;
   a primer set of primers respectively having base sequences SEQ ID NOs: 10 and 11;

a primer set of primers respectively having base sequences SEQ ID NOs: 12 and 13; and
a primer set of primers respectively having base sequences SEQ ID NOs: 20 and 21.

Fig. 1A

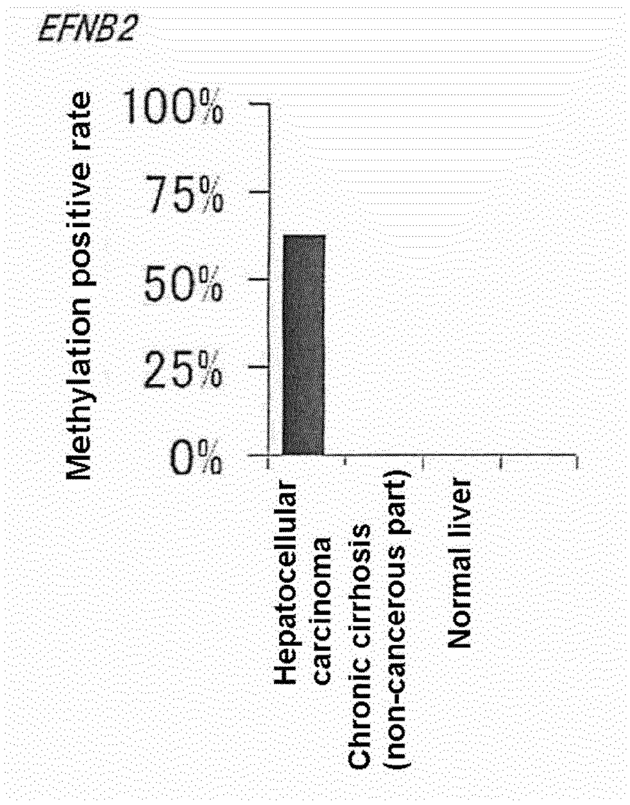
EFNB2
Methylation positive rate
100%
75%
50%
25%
0%
Hepatocellular carcinoma
Chronic cirrhosis (non-cancerous part)
Normal liver

Fig. 1B

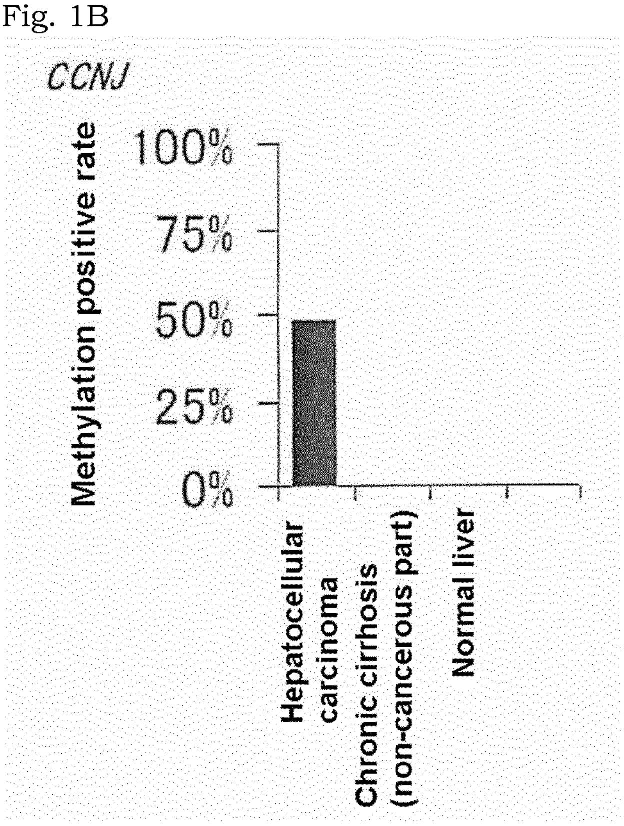
CCNJ
Methylation positive rate
100%
75%
50%
25%
0%
Hepatocellular carcinoma
Chronic cirrhosis (non-cancerous part)
Normal liver

Fig. 1C

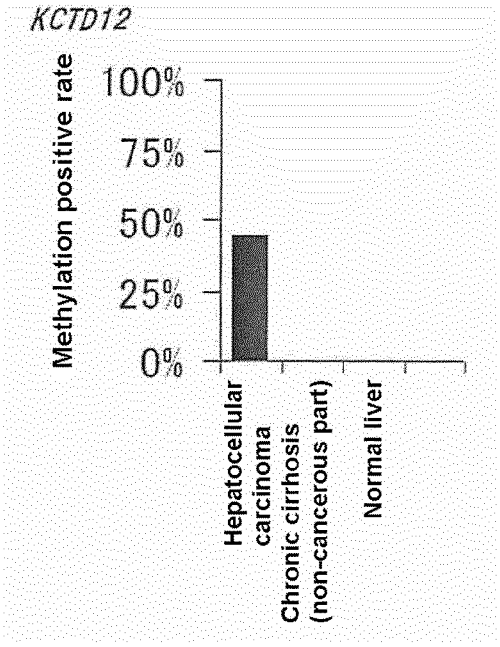
KCTD12
Methylation positive rate
100%
75%
50%
25%
0%
Hepatocellular carcinoma
Chronic cirrhosis (non-cancerous part)
Normal liver

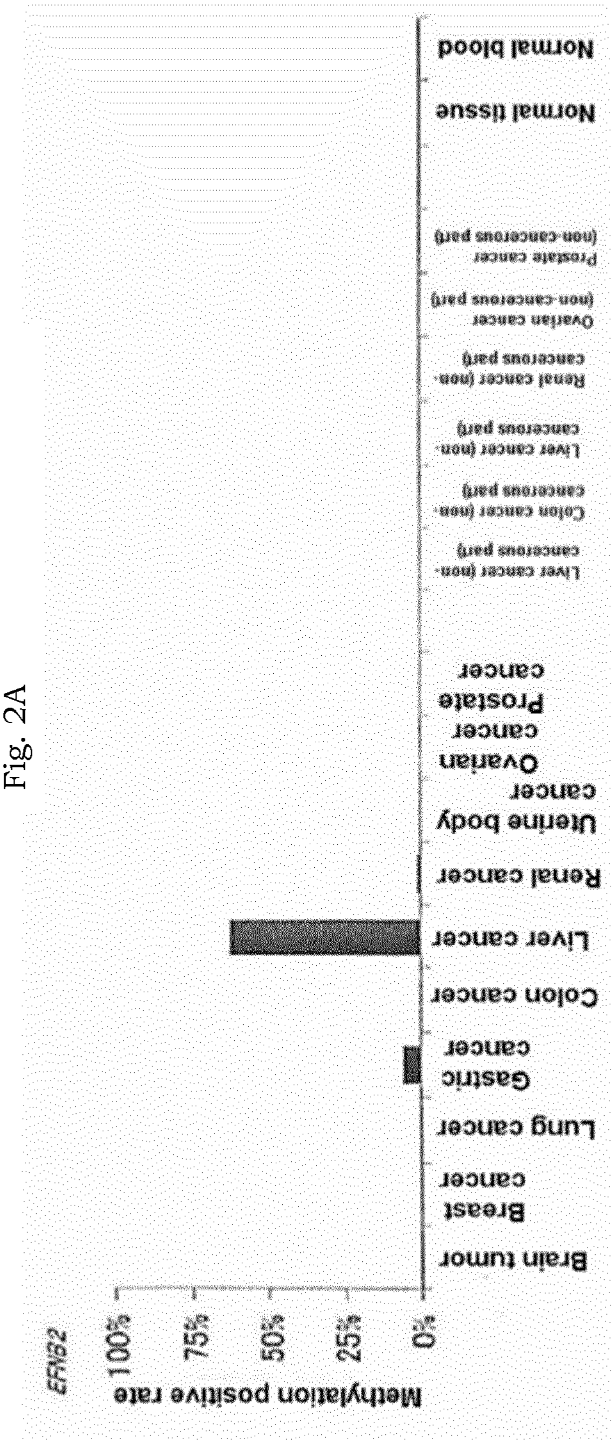
ERBB2
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Liver cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 2A

Fig. 2B

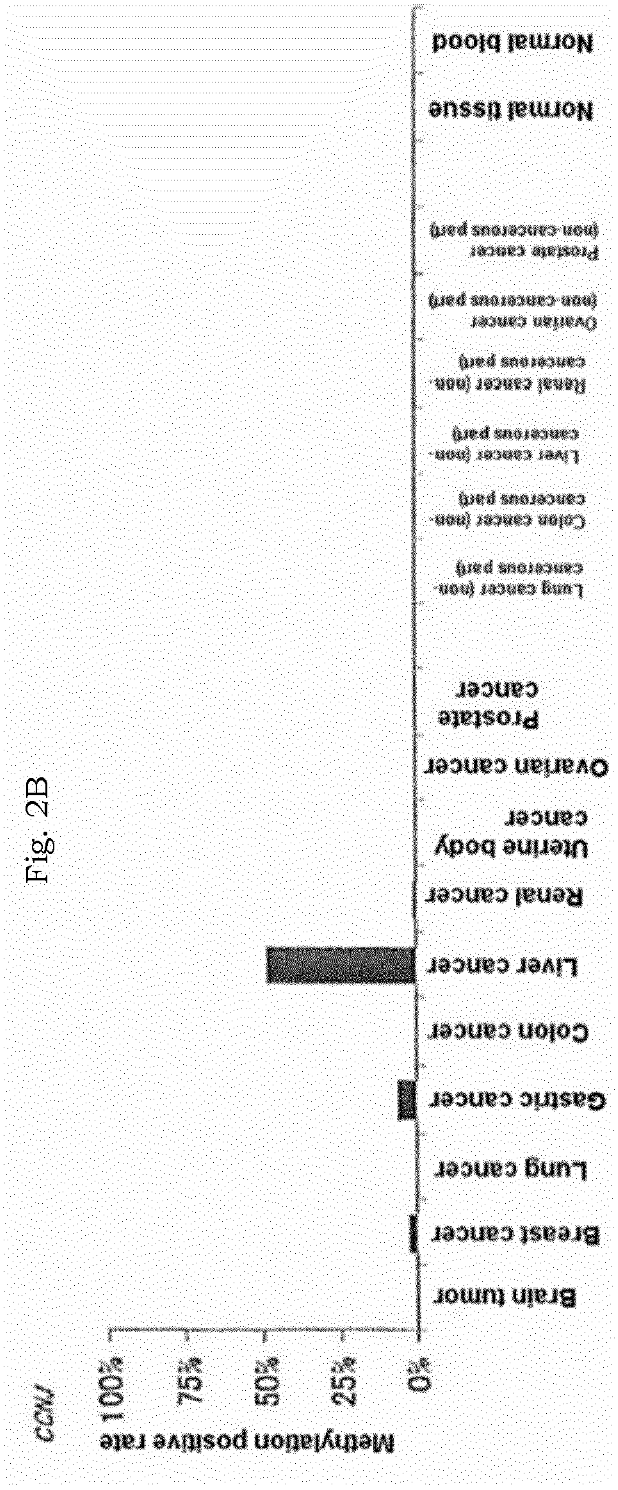
CCNJ
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

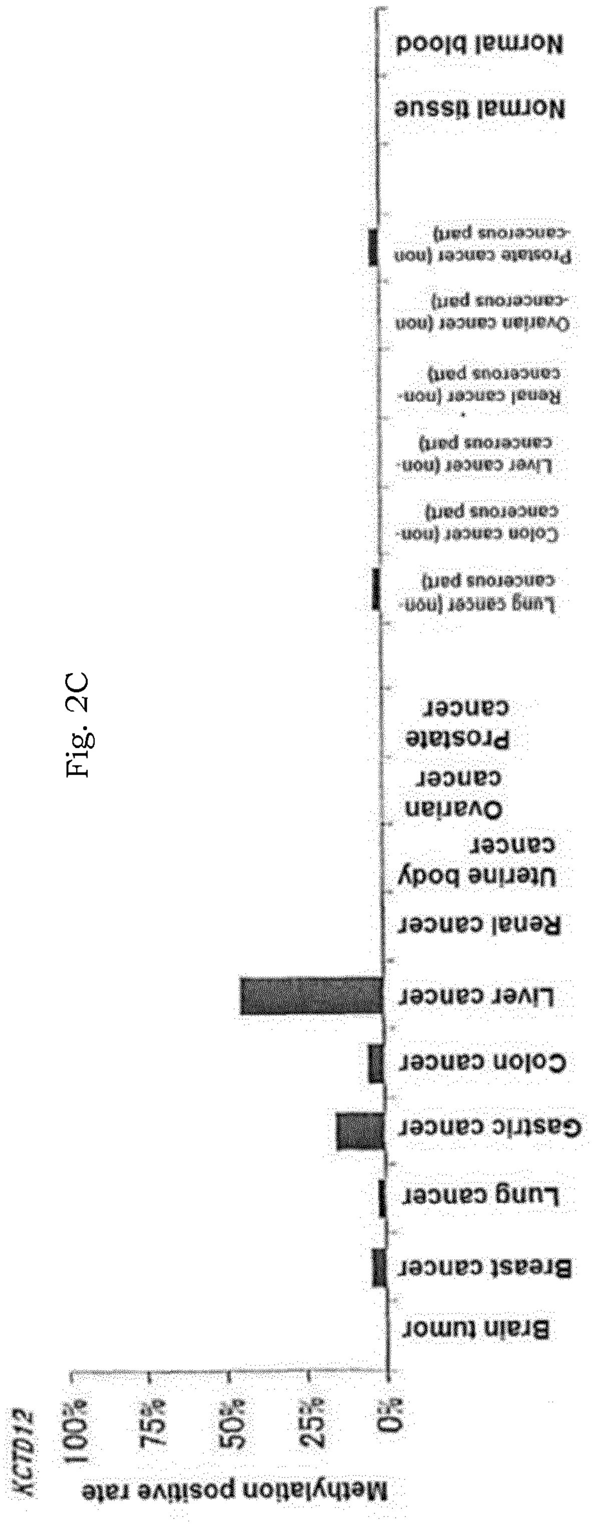
KCTD12
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 2C

Fig. 3A

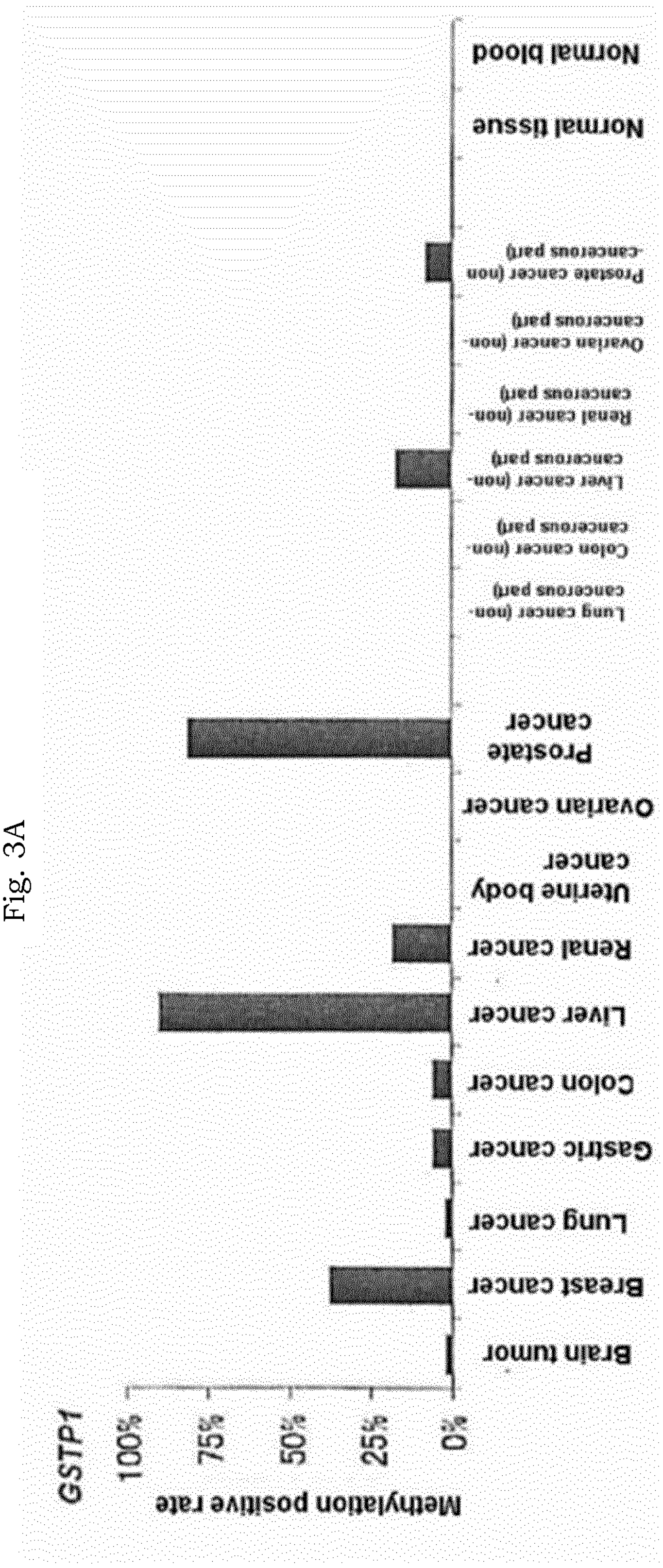
GSTP1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3B

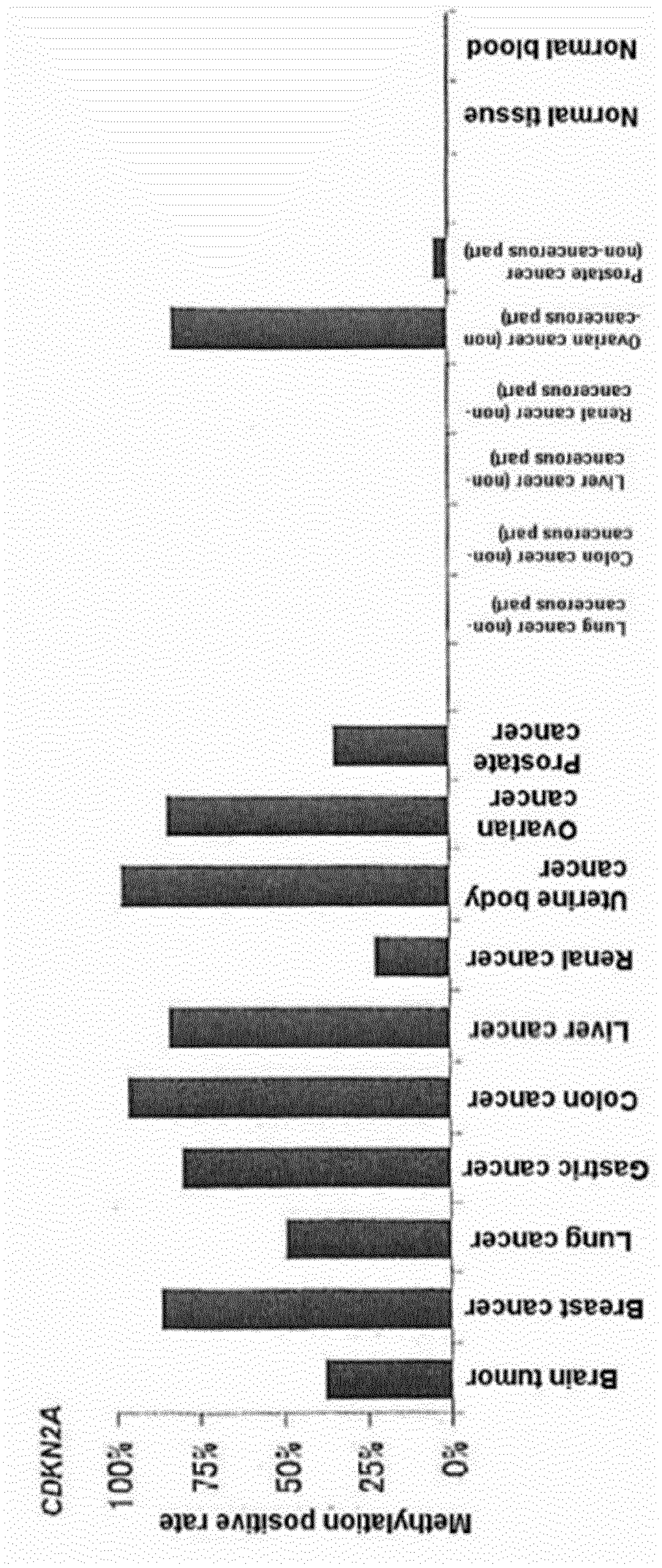
CDKN2A
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Renal cancer (non-cancerous part)
Ovarian cancer (non-cancerous part)
Prostate cancer (non-cancerous part)
Normal tissue
Normal blood

Fig. 3C

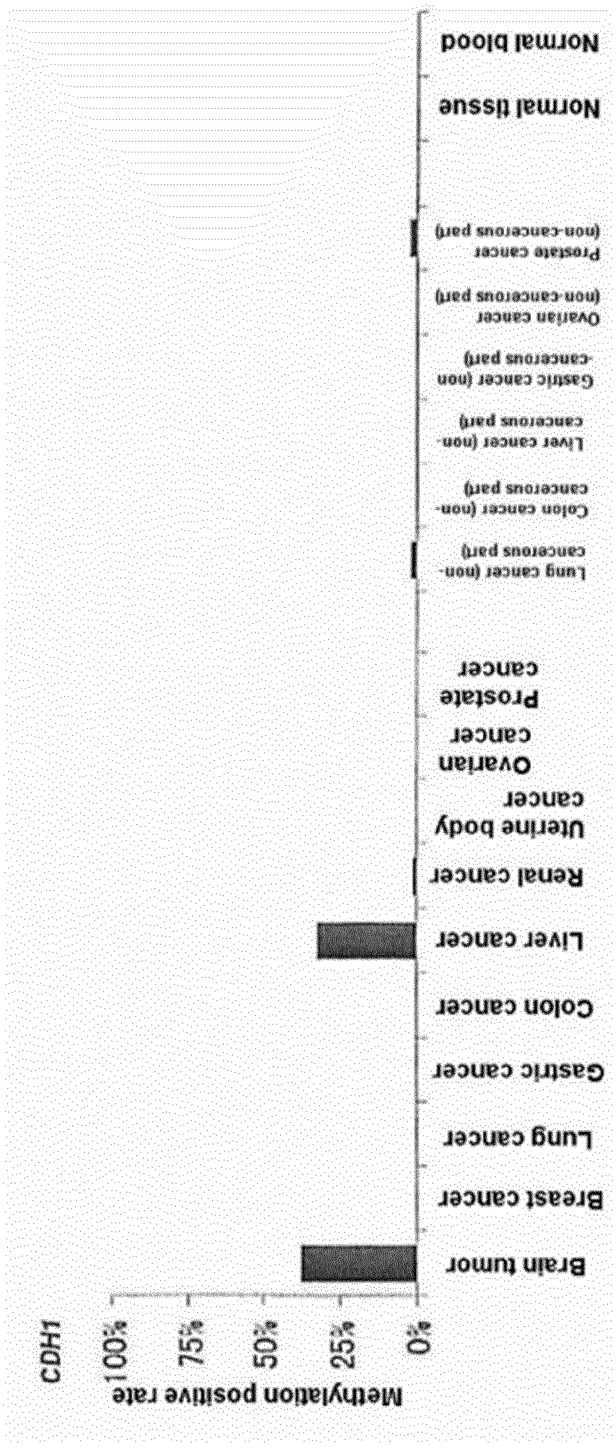
CDH1
Methylation positive rate
100%
75%
50%
25%
0%
Brain tumor
Breast cancer
Lung cancer
Gastric cancer
Colon cancer
Liver cancer
Renal cancer
Uterine body cancer
Ovarian cancer
Prostate cancer
Lung cancer (non-cancerous part)
Colon cancer (non-cancerous part)
Liver cancer (non-cancerous part)
Gastric cancer (non-cancerous part)
Ovarian cancer (non-cancerous part)
Prostate cancer (non-cancerous part)
Normal tissue
Normal blood

Fig. 4

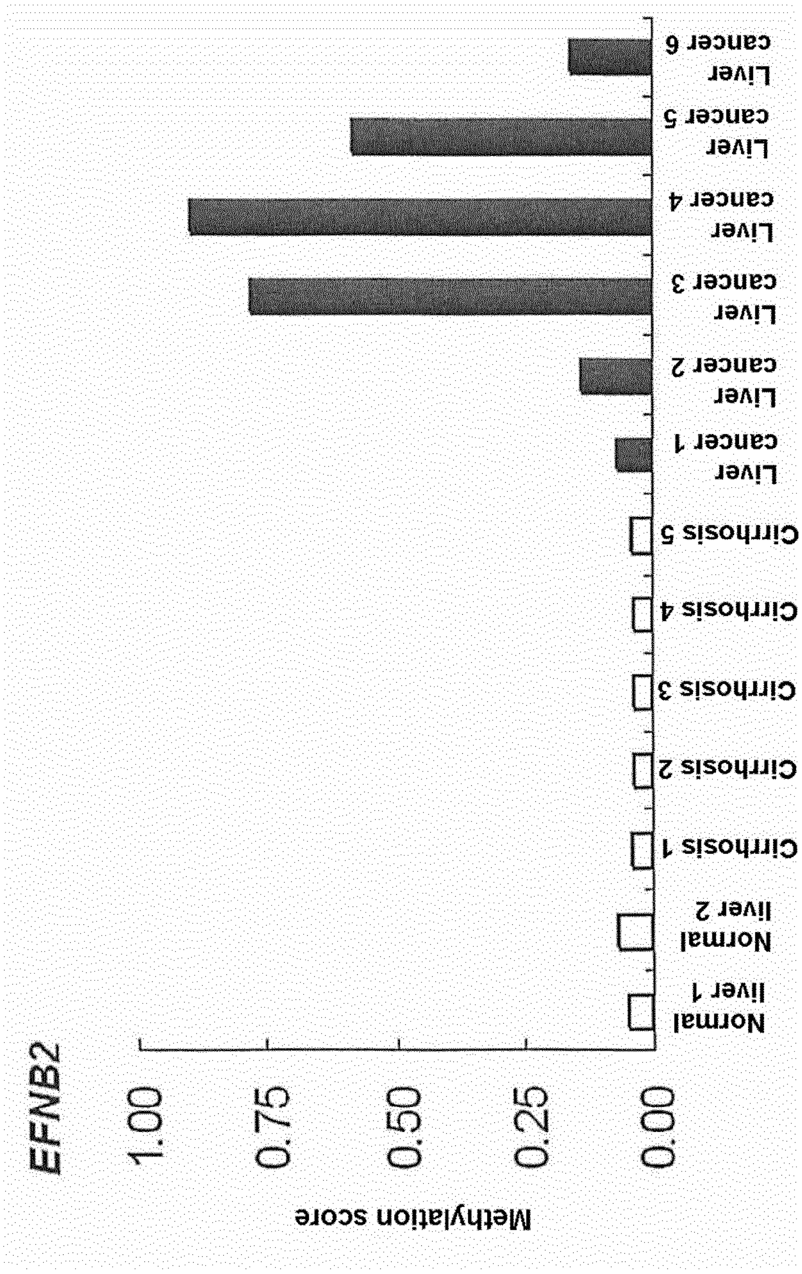
EFNB2
Methylation score
1.00
0.75
0.50
0.25
0.00
Normal liver 1
Normal liver 2
Cirrhosis 1
Cirrhosis 2
Cirrhosis 3
Cirrhosis 4
Cirrhosis 5
Liver cancer 1
Liver cancer 2
Liver cancer 3
Liver cancer 4
Liver cancer 5
Liver cancer 6

Fig. 5

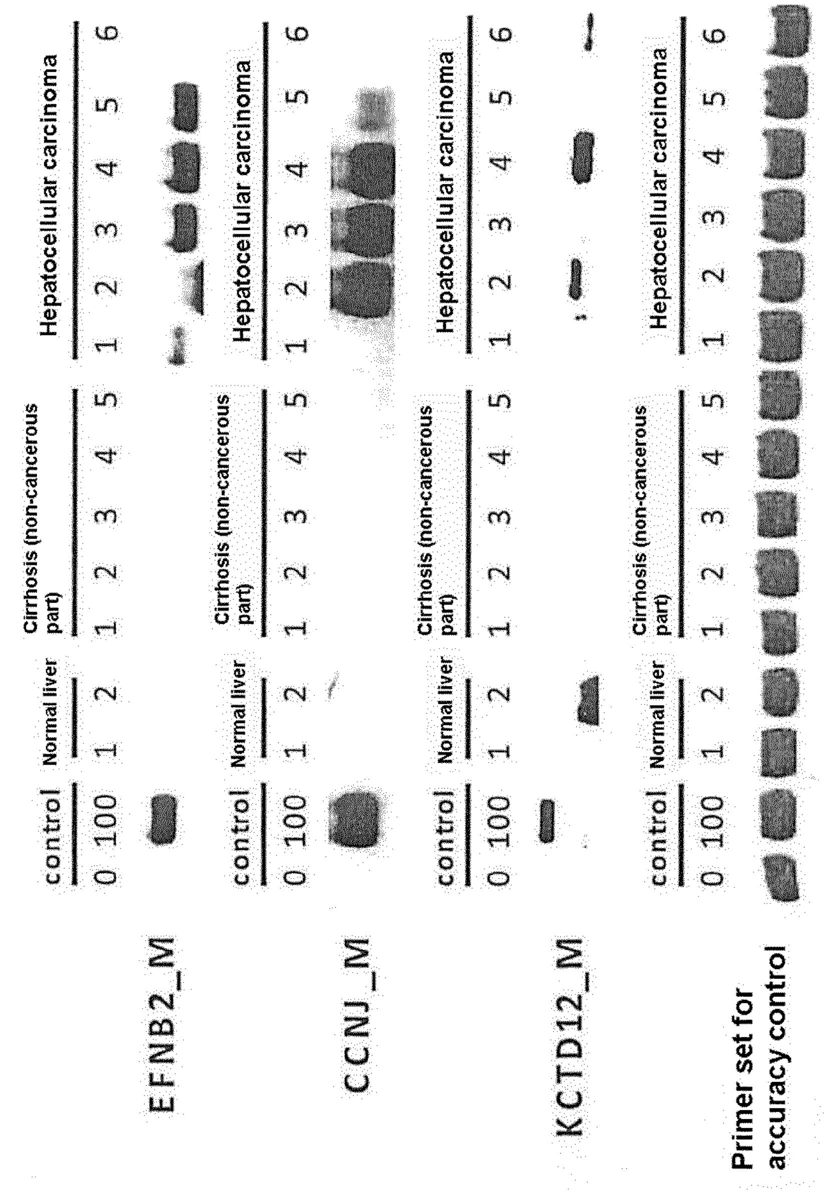
EFNB2_M
CCNJ_M
KCTD12_M
Primer set for accuracy control
control
0 100
Normal liver
1 2
Cirrhosis (non-cancerous part)
1 2 3 4 5
Hepatocellular carcinoma
1 2 3 4 5 6

Fig. 6

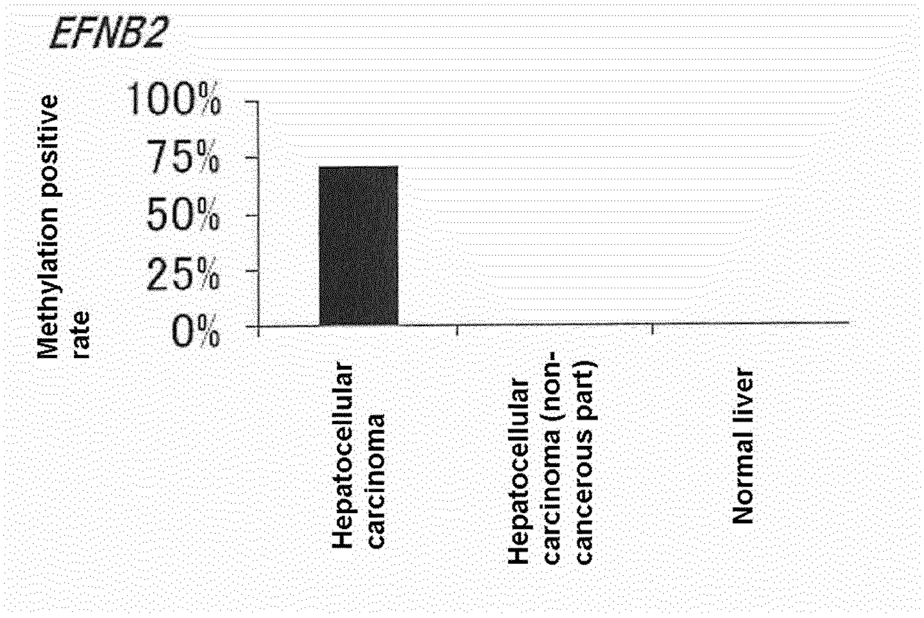
EFNB2
Methylation positive rate
100%
75%
50%
25%
0%
Hepatocellular carcinoma
Hepatocellular carcinoma (non-cancerous part)
Normal liver

Fig. 7

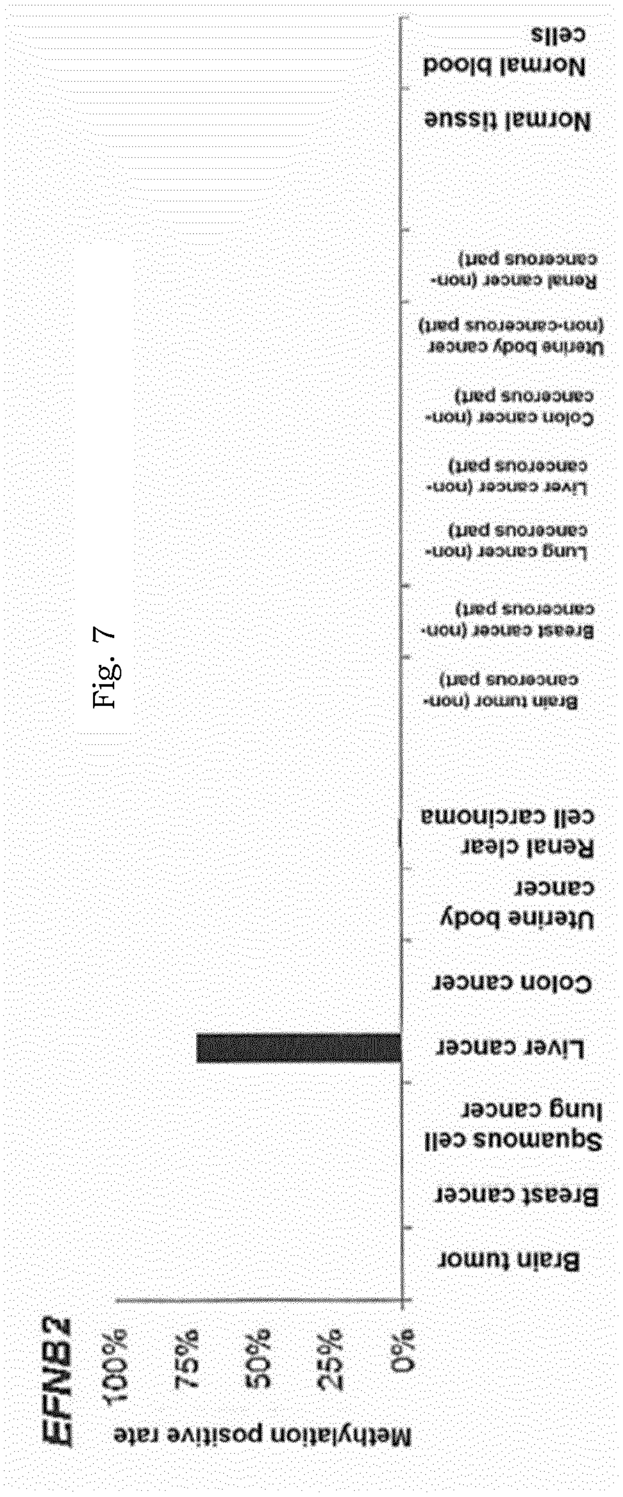
EFNB2
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Normal blood cells

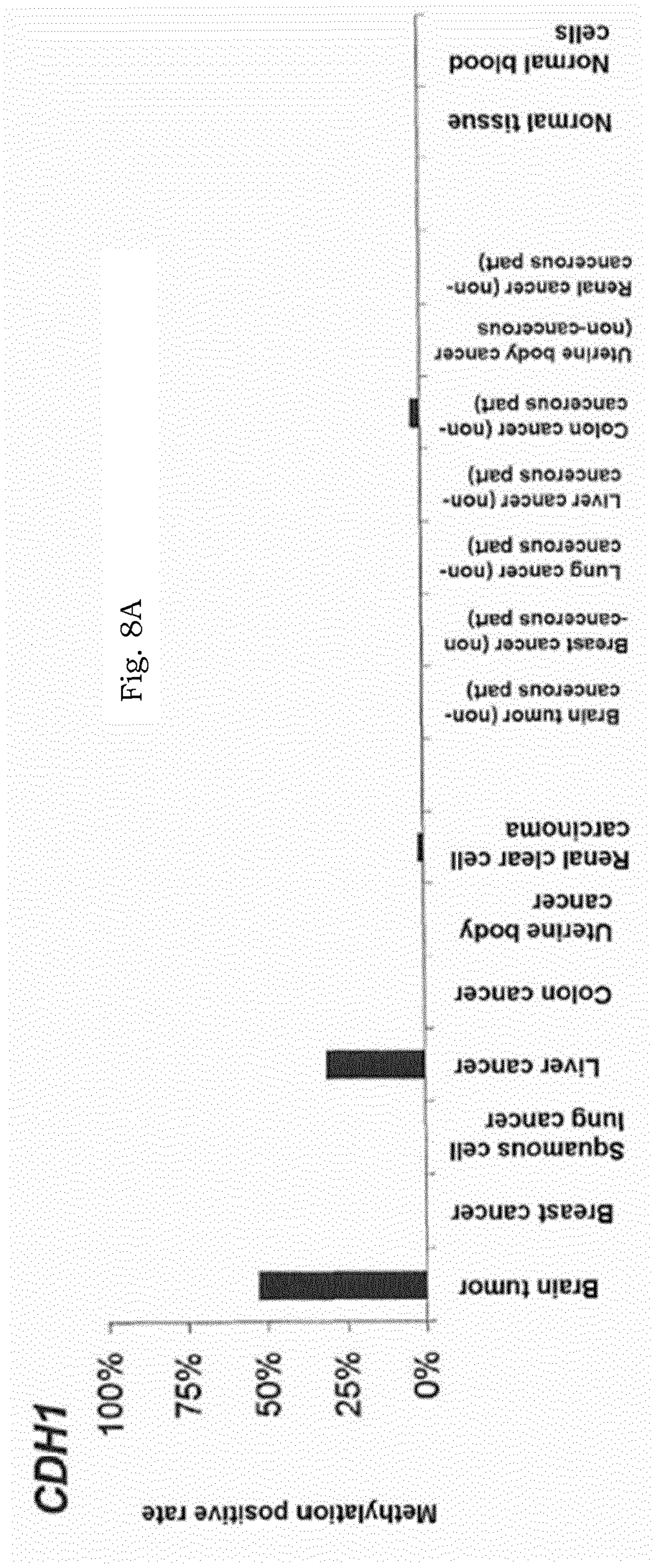
CDH1
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous)
Renal cancer (non-cancerous part)
Normal tissue
Normal blood cells
Fig. 8A

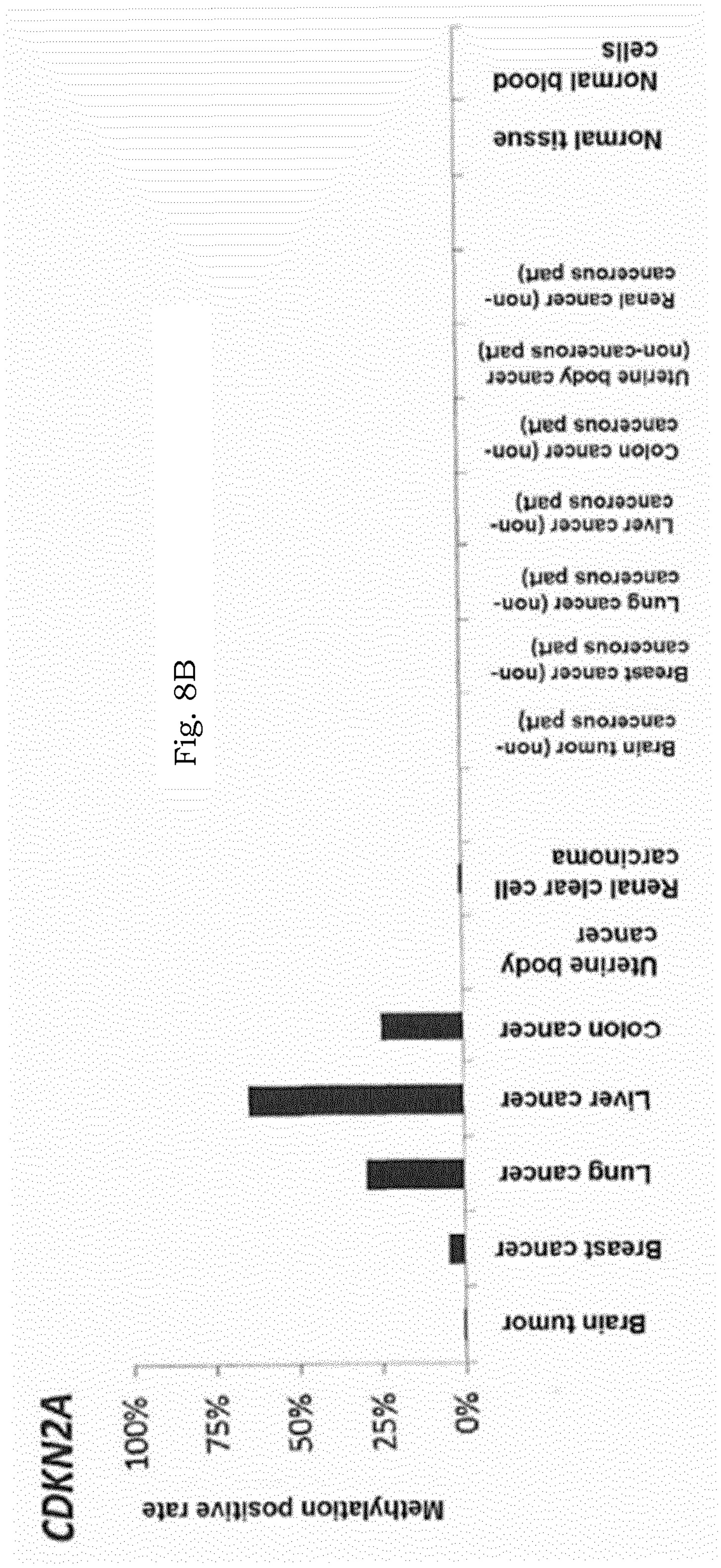
CDKN2A
Methylation positive rate
100%
75%
50%
25%
0%
Brain tumor
Breast cancer
Lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Normal blood cells

Fig. 8B

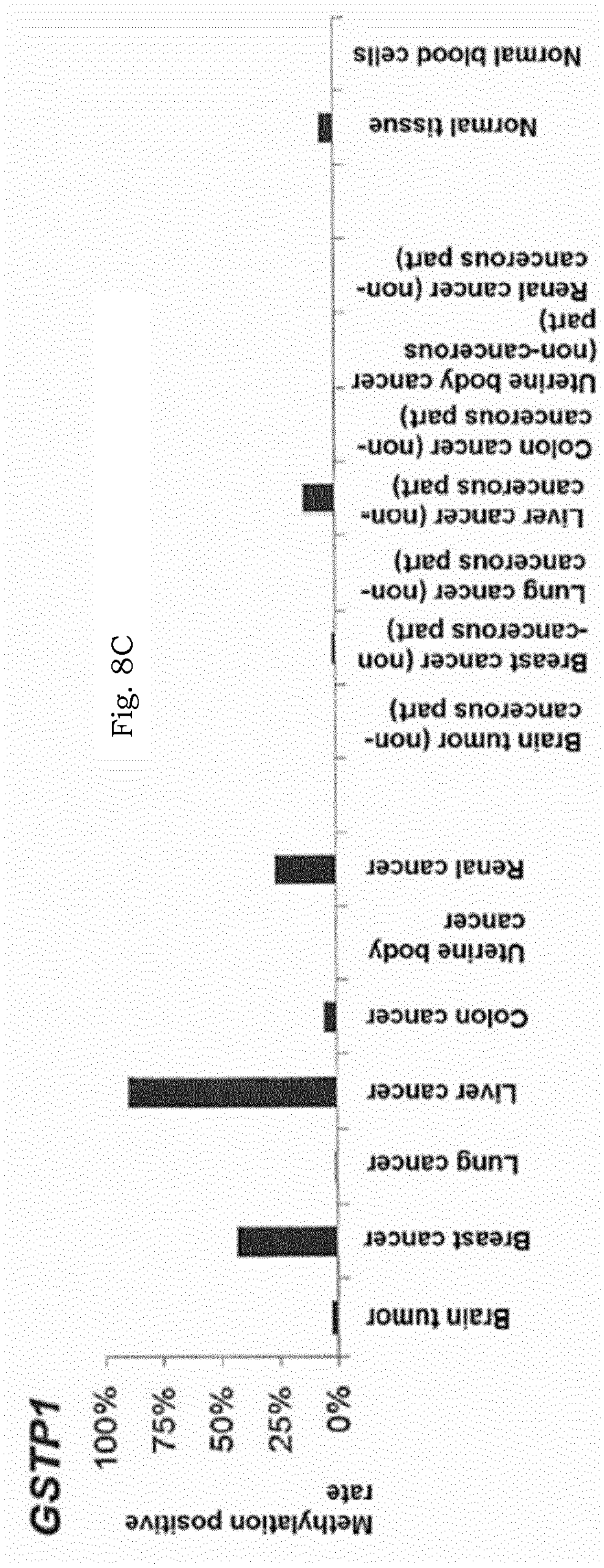
GSTP1
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissue
Normal blood cells

Fig. 8C

Fig. 9

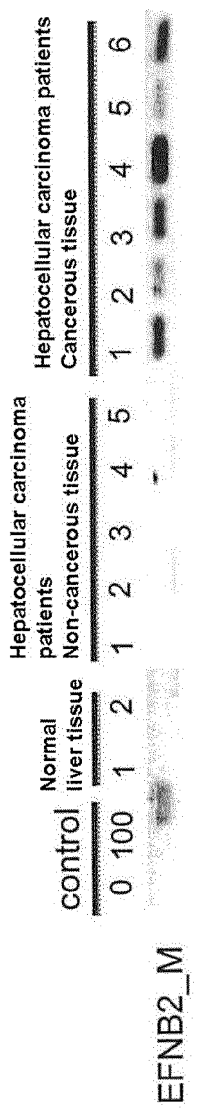
EFNB2_M
control
0 100
Normal liver tissue
1 2
Hepatocellular carcinoma patients Non-cancerous tissue
1 2 3 4 5
Hepatocellular carcinoma patients Cancerous tissue
1 2 3 4 5 6

Fig. 10A

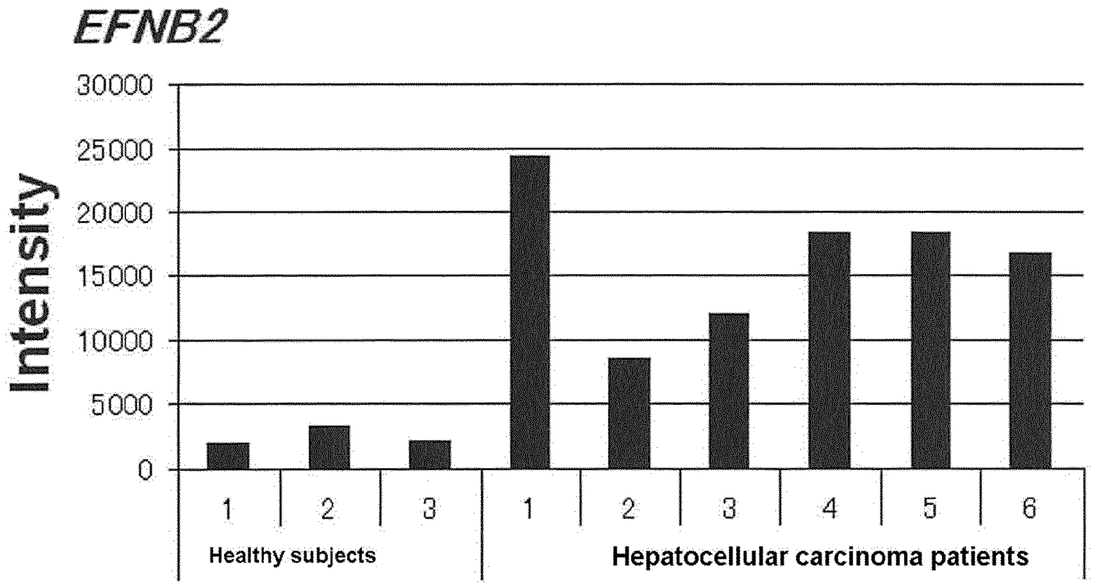


Fig. 10B

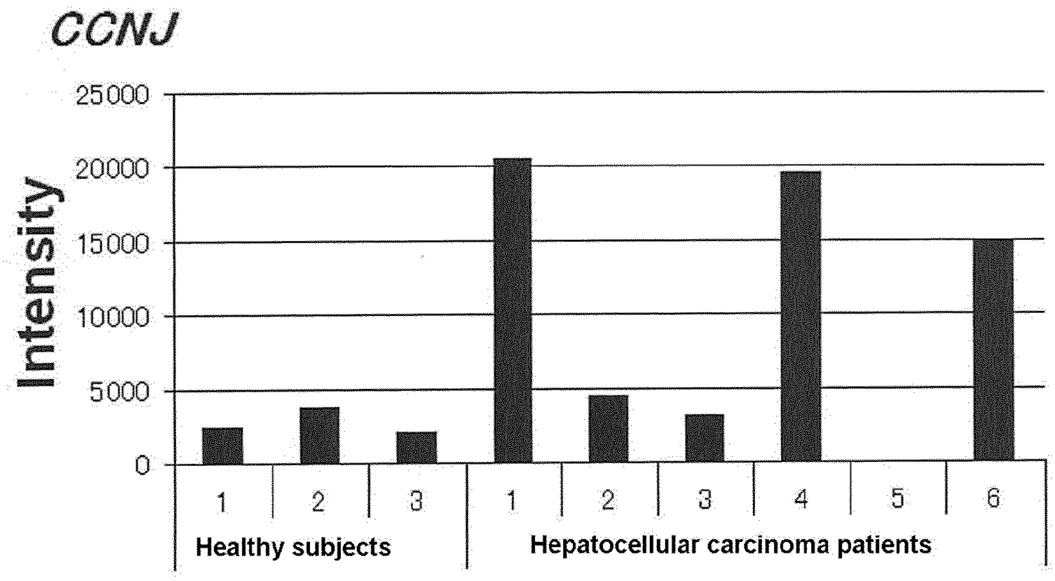

Fig. 10C

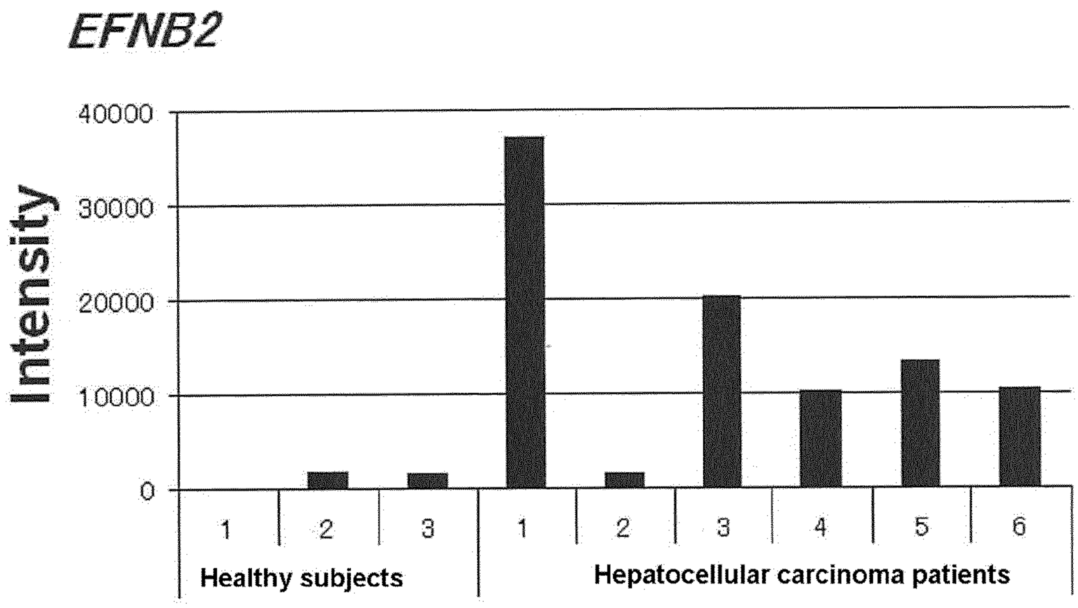


Fig. 10D

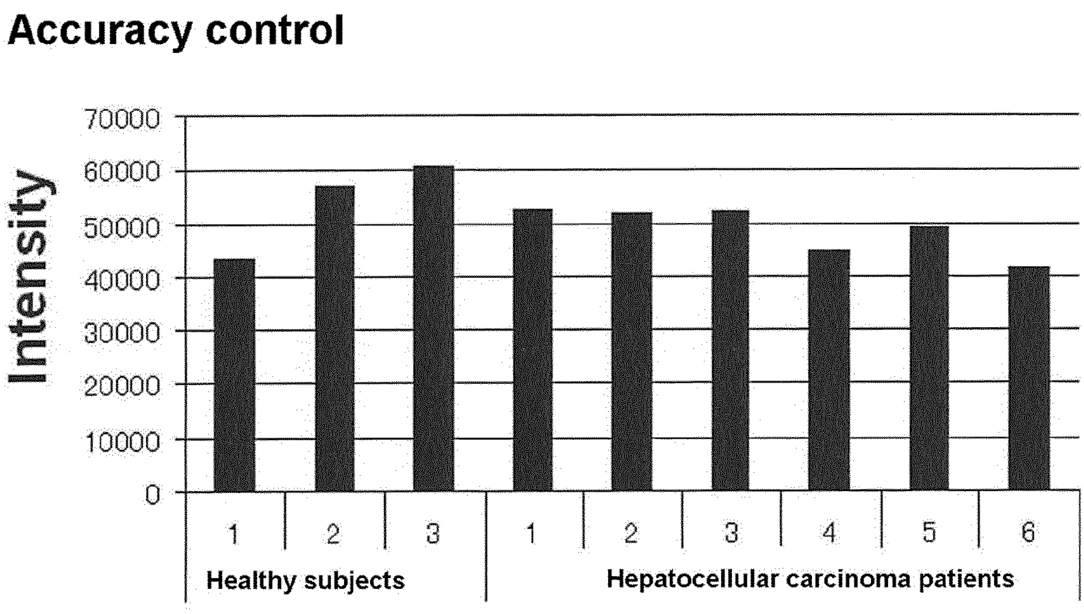

Fig. 11

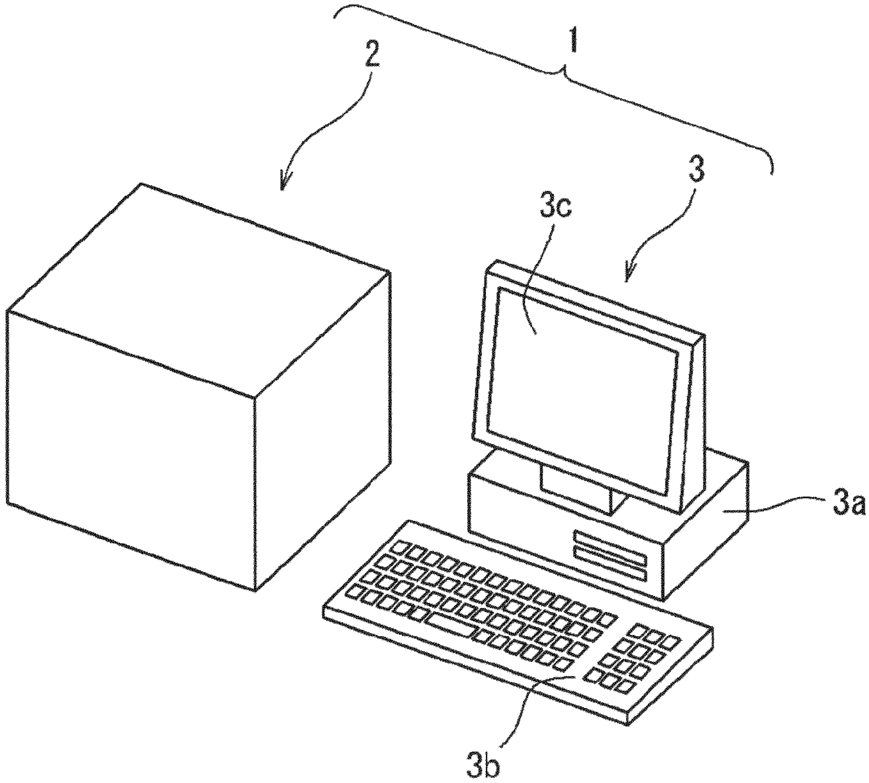

Fig. 12

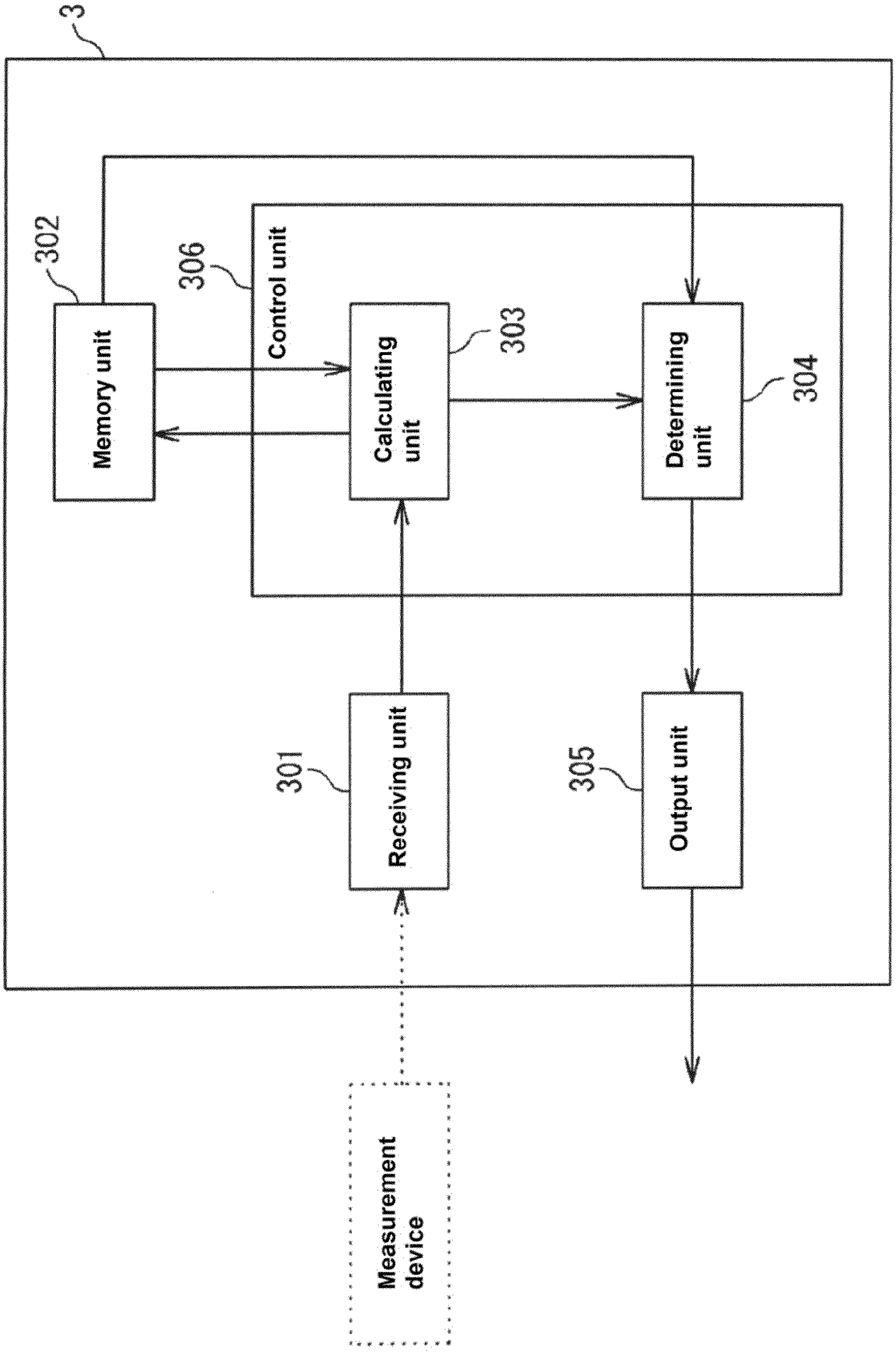
3
302
Memory unit
306
Control unit
Calculating unit
303
Determining unit
304
301
Receiving unit
305
Output unit
Measurement device

Fig. 13

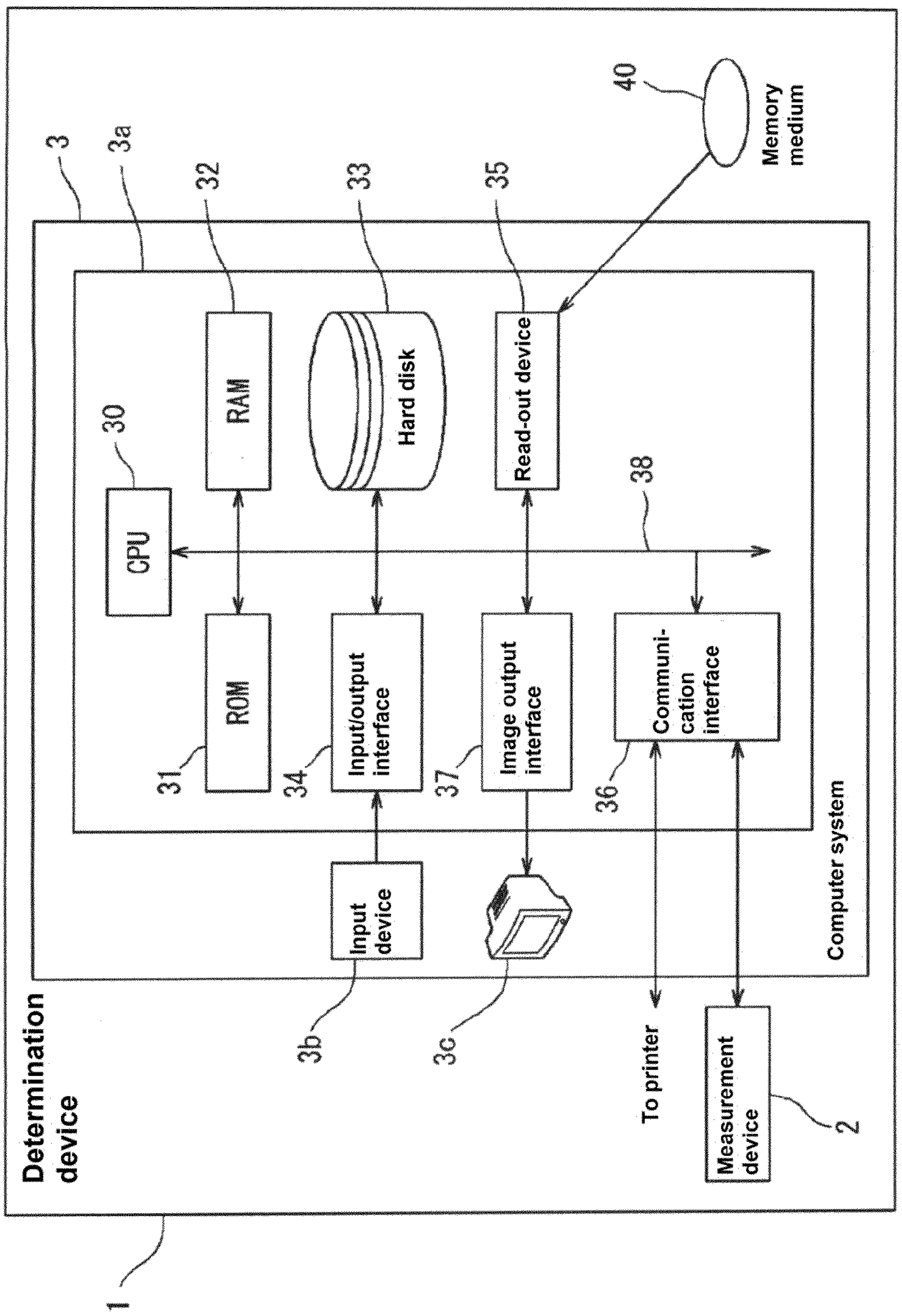
Determination device
1
3
3a
Computer system
CPU
30
RAM
32
ROM
31
Hard disk
33
Input/output interface
34
Read-out device
35
Image output interface
37
Communi-cation interface
36
38
Memory medium
40
Input device
3b
3c
To printer
Measurement device
2

Fig. 14

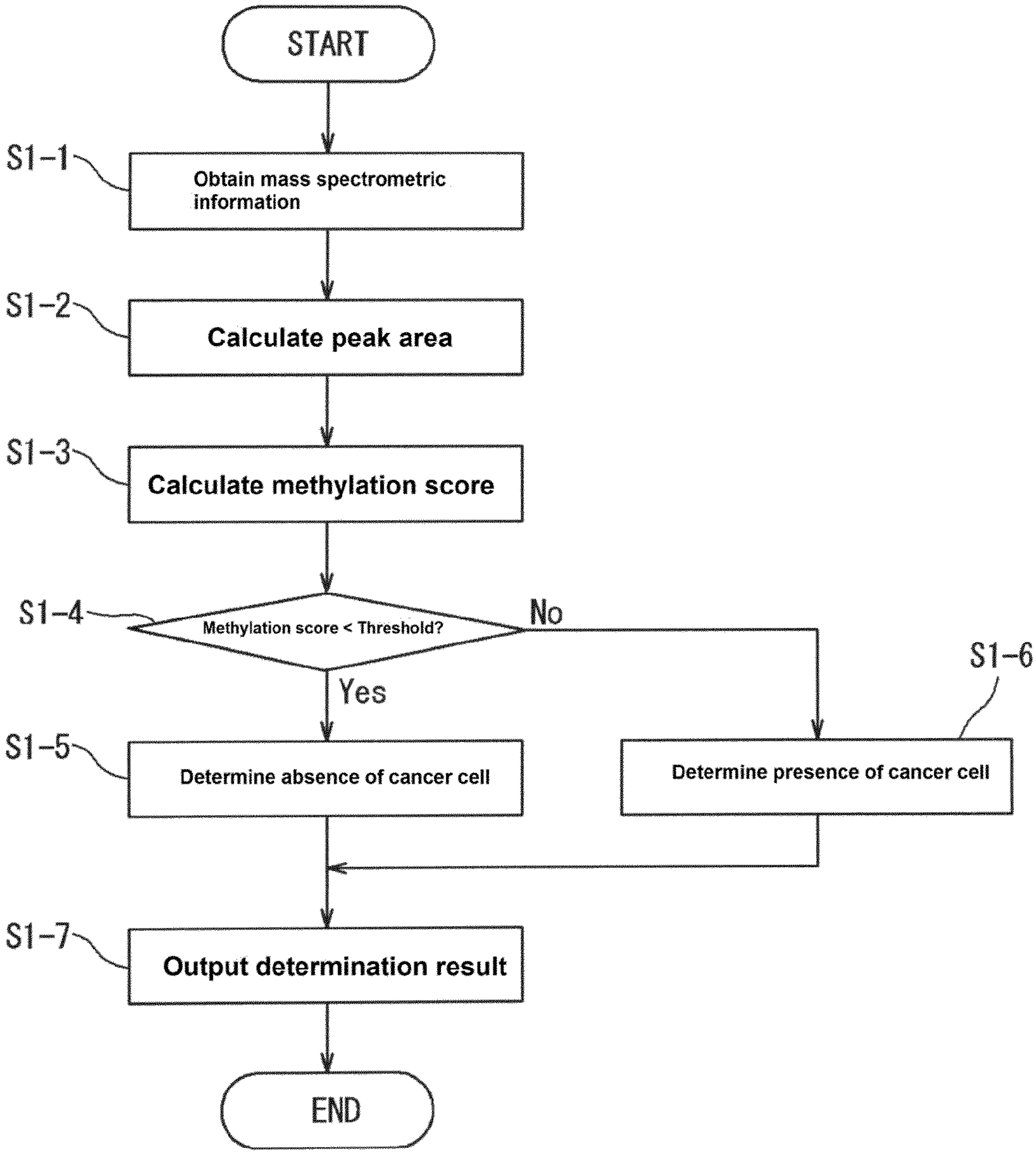
START
S1-1
Obtain mass spectrometric information
S1-2
Calculate peak area
S1-3
Calculate methylation score
S1-4
Methylation score < Threshold?
No
Yes
S1-5
Determine absence of cancer cell
S1-6
Determine presence of cancer cell
S1-7
Output determination result
END

**INTERNATIONAL SEARCH REPORT**

International application No.
PCT/JP2013/075335

A. CLASSIFICATION OF SUBJECT MATTER
*C12N15/09*(2006.01)i, *C12Q1/04*(2006.01)i, *C12Q1/68*(2006.01)i

According to International Patent Classification (IPC) or to both national classification and IPC

B. FIELDS SEARCHED

Minimum documentation searched (classification system followed by classification symbols)
C12N15/00-15/90, C12Q1/00-1/70

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus/MEDLINE/EMBASE/BIOSIS/WPIDS/WPIX(STN), JSTPlus/JMEDPlus/JST7580(JDreamIII), GeneCards

C. DOCUMENTS CONSIDERED TO BE RELEVANT

| Category* | Citation of document, with indication, where appropriate, of the relevant passages | Relevant to claim No. |
|---|---|---|
| Y | FORMEISTER,E.J.et al. Comparative analysis of promoter methylation and gene expression endpoints between tumorous and non-tumorous tissues from HCV-positive patients with hepatocellular carcinoma. Mutat.Res.,2010,Vol. 692,p.26-33 | 1-9 |
| Y | DENG,Y.-B.et al. Identification of genes preferentially methylated in hepatitis C virus-related hepatocellular carcinoma. Cancer Sci., 2010,Vol.101,No.6,p.1501-1510 | 1-9 |
| Y | TISCHOFF,I.et al. DNA methylation in hepatocellular carcinoma. World J. Gastroenterol.,2008,Vol.14,No.11,p.1741-1748 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 October, 2013 (04.10.13) | 15 October, 2013 (15.10.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2013/075335

C (Continuation). DOCUMENTS CONSIDERED TO BE RELEVANT

| Category* | Citation of document, with indication, where appropriate, of the relevant passages | Relevant to claim No. |
|---|---|---|
| Y | ZHU,J. DNA methylation in hepatocellular carcinoma. J.Hepatobiliary.Pancreat.Surg.,2006, Vol.13.No.4,p.265-273 | 1-9 |
| Y | Genta NAGAE et al., "Promoter Array o Mochiita Kangan no Morateki Methyl-ka Kaiseki", Dai 65 Kai Proceedings of the Japanese Cancer Association, 2006, page 254 (O-408) | 1-9 |
| Y | TAO,R.et al. Methylation Profile of Single Hepatocytes Derived from Hepatitis B Virus-Related Hepatocellular Carcinoma. PLoS One, 2011,Vol.6,No.5,e19862,p.1-11 | 1-9 |
| Y | JP 2011-518773 A (China Synthetic Rubber Corp.), 30 June 2011 (30.06.2011), & US 2011/0085973 A1 & EP 2269071 A1 & WO 2009/117096 A1 & CN 102037362 A & KR 10-2011-0037932 A | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LEE S. et al.** *Am. J. Pathol.,* 2003, vol. 163 (4), 1371-1378
- **SELAMAT SA et al.** Genome-scale analysis of DNA methylation in lung adenocarcinoma and integration with mRNA expression. *Genome Res.,* July 2012, vol. 22 (7), 1197-211
- **KOBAYASHI Y et al.** DNA methylation profiling reveals novel biomarkers and important roles for DNA methyltransferases in prostate cancer. *Genome Res.,* July 2011, vol. 21 (7), 1017-27
- **SALHIA B et al.** DNA methylation analysis determines the high frequency of genic hypomethylation and low frequency of hypermethylation events in plasma cell tumors. *Cancer Res.,* 01 September 2010, vol. 70 (17), 6934-44
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), 41361

SEQUENCE LISTING

<110> SYSMEX CORPORATION
THE UNIVERSITY OF TOKYO

<120> METHOD FOR OBTAINING INFROMATION ON HEPATOCELLULAR CARCINOMA, AND MARKER AND KIT FOR OBTAINING INFROMATION ON HEPATOCELLULAR CARCINOMA

<130> TM5778PC

<150> JP2012-205756
<151> 2012-09-19

<150> JP2013-113430
<151> 2013-05-29

<160> 24

<170> PatentIn version 3.5

<210> 1
<211> 4079
<212> DNA
<213> Homo sapiens

<400> 1
cttaagtcta gaacacagtg aagtaacttc ctctgctgaa ataaaatctc atttgatgag 60

ggtttaaata catctgctaa aagtatagat ttctcgaaat acattgatca tgattcaaag 120

tgtggtaaag aaaaaaatta accaagtttt cccaataatt gtaaaatgaa gtccaaatac 180

caagagatac gacactgatg tgattggttc ttggtttggc atgctttgga aattctactt 240

tgccttgtgc atgccagtat gacctacttt tttgctggca tgttatacaa tagtagatac 300

aaatggcttt gaggtgatgc ttttaattac caaaattacc attaaatagt agaatgttga 360

catcttcgaa gtgagtactg ctgatgcttt tcacattgtc tacattgaat ctctgttgac 420

tgaaactaag ctgtatattc tctcacacta tagatgtttt gtgtgtgtgt aggattcctt 480

ttcagtcaaa tacgcacttt gttttgaaaa ggaagcgatt ttaaagtgta tatttacatt 540

tgtcccaagg taaaacgctt tggatgtgag actttctgat ttactgaaaa tgttattttt 600

ggaagctggc ttattttcag acaatgcaaa tttcctgtat ttcctctagt ttacttggag 660

aggaggatgt agtgagctaa gaggtcacaa tttaaatttc acaatataag tttgtcaaaa 720

ggtactgagt acccagtgtt cttttaaaag atcattaagc taactttata ttcaatttca 780

atgagttcct taaatggtat tttagcatac taaaaactag ccctttctca cccttgtcaa 840

ggtaggagtc atagtgcctg ctgcttcctg ggttgttggt gcttcctggg aatccgctgt 900

tcaaatcgtc ataccgtggt ggagcctttt taaaataaat tttatatata tatgtgtgtg 960

tgtgcatata tatatgtgta tatatatacg tatatatgtg tgtgtatata tatacgtaca 1020

tatgtgtata tatgtataaa tatgtgtata tgtgtgtata cgtatatgtg tgtatatata 1080

tatttagttt gaaggcttat ttaaaatgaa aataaattat gaaacttttc tgctctcaaa 1140
gatctgtttg gaagagtaag tgtattcagt actttgtatg ccttaatgtg ttttctatct 1200
taaactagtc ttcacaagct tttaccactc caactgagat gtatacgtta agttagacta 1260
atgtttcaaa aacggatact taaaagcaac gtaccataaa aatcttcaga aattgttaca 1320
aagaagagtg aaagaaaaca aggtgtttta ccatatgcat ttttaaattt agcgtagtgg 1380
atactttgtt ttaaaaatac ataatttgta gaattactgc ccagtttgtg tttgaaacat 1440
aaatcctggg gtggacggca ttaaattgaa aaaaaacgtg ggcatacagt atattttaat 1500
ttttcatact gaatcgtaca tttgcactgt gccttatttc taccgtgttc ttcctactgt 1560
tggacatata aaaagatttt ccttggaact gtgacttgat tagatgctgc agtatgctgg 1620
atccgggtcg gtggaccgca gagcgctcga gatgcggaga agcaaagctg gccgctagtg 1680
ggcgctgtgg ctccgtggaa gtcacggacg agcgtcagca ccggcgctcg accgggaaag 1740
tagcgaggag ttcctgccag ctctgctctg ggtcttgca gacgccgagt gagaagcaca 1800
tgccttgact tttccatctc acaccctcta aatctctggt cactttggga agcgcgcgtg 1860
tctcctagag gctagtgaga cggtccccgc gtcgtgcctg tgccggcgac acgcggggaa 1920
agctccaggg gcgccacctt cccgggggc caggagggaa gggagacgcc cgcagagaag 1980
gcgggaggcg acgatgctca ggccctgatt agagctgcaa attccggcgc cgcgtcgcag 2040
gaatcgtctt gcaattcccg gccccactga ctgaaactga gccgtaggcg attggtacca 2100
aaatcaagtg agaagccctc gccgagaggc gtcgggagaa gcgcgcagaa gccgggagct 2160
ctaagggtgc gcgcggagtg caagtcttgc acgcggggca gcgccgcatc tgcggagaag 2220
ggacgccgag cggagcctgc tgccttccgc actgctcgag ggaaagccgc gccctctgat 2280
tccgccgagg gggaaggcgc ccccgggcgc ggagacaggt ggcccgctac tcggccgtcg 2340
ctgtttccac gtctgcaccg cgcccagaag gcctctcgcc gccccggggg cgccctgtgc 2400
acgcgctgag cgcttctcct gcccggcttc tgctccgctc ccgccgcccg cgggagccag 2460
gggcggggct cacctgtcag cccgagcccc gcctcgcggc acccaatgtc gggaggggat 2520
ggcggacgga cggaccggcc gtccaaccag cgcgcggccg cggagccgcg ggccaatggg 2580
cttcgcgcgg cggggcgggg ccccgcgttt atagcgctct gacagcgcgg cggccgcgct 2640
gactctcctc ggcggggacc gcggcgccgg ccggagcgag gagctgcgca cgcagcgggt 2700
cccggcgccc tccccgcaca caaaggcccg cgcgcgtccg gagcccgcgg cggggaccga 2760
gctccctctt tcctgccctg gggggccggga gccgcgcgga ctgagaaggc tcctgcgcgc 2820
ccggaggcgc cctactccgc tccgtgctcc gggacatgga accgcgccga cgcggcgccc 2880
gcgcgctcgg gccgctgccc gctgcactgg atctatagtc acaggcggcc ccgctcgggg 2940
cgcagcgccc gccgcccgcg ccggtcgtct ccgctccggg tctttgtgtc ccagcccgca 3000

cccgccccgc gcccacccgc cagccgcggc cggcccggca ccctggagcc gcacgggagt 3060

cggccgtccg agctgcgtcc ggcgcggcgc cccggaaccc cgagtccgcc gcgccgccgc 3120

gccccgcgcg tgcgctcccg ccccgcgccc tgagggcccc ggagagcgag cgcacctggc 3180

ccggcgaccg ctggagctgc gcagcgcgcc tcggagctgc ctgcgggcgc acgccgtctt 3240

ccccgccagt ctgccccgga ggattggggg tcccagcctg cgtcccgtca gtcccttctt 3300

ggcccggagt gcgcggagct gggagtggct tcgccatggc tgtgagaagg gactccgtgt 3360

ggaagtactg ctggggtgtt ttgatggttt tatgcagaac tgcgatttcc aaatcgatag 3420

ttttagagcc tatctattgg aattcctcga actccaagta agtggcgtcc gcgatccccc 3480

tatgtccccg ccccggggtc cgccgcgccg tccgggcggg aggaggggtc agtccgcggg 3540

gcctcggagc ctgtttctgg aacctcggtt ccccgtcccc cacccccaac ccccgcccca 3600

tttcactagg tggagactcc tcgctcggct ttccaacccg agccccgctg gaacggacgg 3660

tctctccgcc tttcctcccc cgaacgctcc caggcgctaa aagctactat cggctcgggt 3720

gtcaagtccg ggaaggtgtc cgatggcgat acctgaccct ctcctgtttt cgaggacgaa 3780

ggacatggcc acaatctagg ctggccggca cgcggggact ggtgggctct ggagagaggc 3840

ggagatgctg cattcgcggg gagcgcgggc ggcgtggtcc ggggcccgcg ggcgggcgac 3900

cggggtggca ggacgctggc agcgaagcgc gttctggaga ggggagcctg gagtcgctac 3960

gctgcccgca gagccctgga gccggggcgc cttggcaccg cgccgccagc ccgagggtgc 4020

gcggggagct cgcctgcttc gcaggagaac tcgggcgtcg agccctttcc tccgcgccg 4079

<210> 2
<211> 4093
<212> DNA
<213> Homo sapiens

<400> 2
aaaaagtact cttaatatca actaattaat ttcactacat accagaggga taaaaaggct 60

gacacttaaa acacttttca aaatatgtgc aactatatta attttttttaa aaaagaaaac 120

atttcacatt gaaagatgac atctgccaaa aaaaaaaaag atcaaaagtg tatctggaca 180

attttccaag aaactgcaac tctttcctaa ctaacactcc agagatccca ttagttagaa 240

tttttatgtg gtataaacca tctcctaggg ggctgaactg tattttagtg aatctaagag 300

actgaatgtc agaattagtt aagtagcatt tagaaatctc aggcctagaa aagggtaaca 360

atttaagtca gaattaggtt ttccaatcta cttgacctgg tcagaaactc aagataaaag 420

ttcattatca ttccaggatc tcaaccccaa gagtcagaat ccttttttatt gaaagtcagc 480

tgttttgctt ctttctcaat tttctacatt taacaaatgg ctttctttat ccaaggtgaa 540

actgagttaa gctagagatc attttggatc tggtctctct tttggttatc gaagaggcag 600

aaatgtctca ttaatctgcc ttgccacatg cagagtaaaa ccgttactta atagcaacaa 660
tacacagact ttgctttgtg ttggaaaagt atttttttcc aatatctatt aagtctactt 720
tctgcctttg atatcagcag gaaggggcag ccaactccct ccactttagg gcttttgaac 780
agcccaaatc ctgaggggta agtgataaga agttatcact tagtgcttac tatataccaa 840
gagcagtttc taagcacatg tacggtacta acttaagttt cacaatcgcc agaatcataa 900
ggcacactca ttattcctca ttttacagat gaggaaatca agaaacagaa atttcacttg 960
tccacgtctc acggcaagtt agcggaaagg ctgaggaatt tatgtacaaa agcgacttaa 1020
actggctgca tgcaacggca actctcgaac aaagagggct gttctgccca acaaaaattc 1080
agtgtatata tcttttaaaa tacaagatca aattcatgac ccacctggaa gccagaacgc 1140
ccctttccac cttccctggc ccaactacca tctccctact cattcaggtt gcaggttctt 1200
ttgatacatc ccagagaagg agccaagaag ttaattaacc ttgagcaaat gagcaaactc 1260
gccttttctg gcccggaaga tagtgagtaa agaacctgac tagaccttct agagagccga 1320
gattcagaac aactggcgcg acacacagaa ggagaaggca ggccgggcct cggagttacc 1380
ttgtagcgaa gcgcttggtg aatatcggcg gccagctgtc ctcgccacca ctgcccctcc 1440
agctccatgg gacccggcgg cgcgcccagc ccgacgcggc aactcgagtc tgtaagacac 1500
accccaggcg cggtcactgc cggctccccc gcgaccctct gcgcccccctc cccgggggt 1560
gtgacctcag gccacccggt gtggctgctc atcttcactt gactcttcct caacccagcc 1620
cgacccgagt cgtttccccc aacgccacac gcgaagccgg gtctgccccc gcaggaagag 1680
ccgaaggact ctgagactca ctgctcgact atgtgtcggg caaaagggga tccactcggg 1740
ccctgagttg ccaaagcgcg gagccaggtg cgagctcggg aagagctggg ggaggcccgg 1800
ctggggctcc ctgcgtccgg ggctggagtt ctcccgccct ccctgaggag cggggagagg 1860
tgacacgctc atagccccgc tgaggggaaa cgctcccatg gcaaagcccg ggggtgagcg 1920
tctgcctggc cgcgggtctc cgcatggacg tcgggagcct gctcctcccc gggagccccg 1980
agaaacgcac gttccgcgcg gcctcagaaa ggggaacaaa aggccgggcc agccaggctc 2040
tgcgcctcac cccgggaggg tccgctgccc cacgcctacc ccaggcctgc cgggccgccc 2100
ggccccacgc tggctccctt acccggaacc gcggccctca cagccccagc cccgccgtcg 2160
tgcggcggac gccggccccg ctcatgctgc ggacagtggg ccgctaagcg ccggcctcag 2220
ctagagccca gcgcctggac gctcgcgccg cccattgtta aaggaccggc aggcggctcc 2280
gcatacaaag ccagcgcggg ggcacttgag gccccgccgc tgccaatctc gtctcgcgat 2340
acagcggggt cgccccgagc cgtccaattg gcggccgggc cgggcccagg tgcagcgcca 2400
ggcggcggcg cggcggctcc cgcctgggta cggtcagcat cccgcacggc gcggcgtggc 2460

ctgggctggc ctggcgcttc ggttgccggg gcgctcagcc gccacctact gcgaagccgg 2520

cctgatcgcc ggctgaggtc ggcagagagc gccggagaaa ttgtaaggtg gatcactgca 2580

gtgaccaatc cagttaggcc actcacctgc tgagaacctt cgatggctcc tcgatgcccg 2640

cggagcaaag ttccgacgcc tcagtttggc gtttaaaagt cttcacggca tggattcggt 2700

ttaccttact tcacgttgca ccaaccgctt ccatcccagg ggctgctccg tggatcatca 2760

ggccctccaa atgtgcttta gctgtttctg cagcccctga agcgtttttt ttttcctgtg 2820

aaattatgct ctttttttaa gttccacctc aaactccacc tttcttctag aaacctaatt 2880

aatctcttcc ccctccttat tttcgttgct gtagggattc ccaagagctt tgaatgaaga 2940

catttaagat tacttctagt aaccttgctt gtcaaaccca tggtggatgt aattaaccat 3000

ctgtgctcgt aaatcaggaa actgcaaaac tgtgagaaca ctccgacgtc aattgaggtg 3060

atatgggggc tgaaatgtga ttagaggaaa cctaatcaat tgtattttaa cctctttggt 3120

ggattatcac aaaagaggtg gatggtaacc ctaggacagt gagactgtcc acttttctga 3180

gactggagat caatctctta atagtaaaac gtaaatattt ttaaaacttc accccttttta 3240

ctttgaatcc tttgtccaaa aagatacatg atgataaaga actactacaa agtgataaat 3300

acattttatg tgattctggg tggcatcttc atttgaagat catcagcaga catgtatatg 3360

agccgtaccc tatccataga cgatgcttat acccaggaga gcaggcccgc aggaaccaaa 3420

ggcaagaaaa gggaaactca gaagctgaag ttggcagatc ccggcctaac ccgacatagt 3480

ccagggagaa ggctgccctg gcctgtctct cagttgcagg gtcctctaga gtgaaggtag 3540

acacatcagg ctaaaagctt catgccattt ttaatggcat ttaaatagtg tctacatcct 3600

tgggttccat ttcatgacta ttaaacaact ttataataat ttggcttcta aataactgtc 3660

cagggcctat ttaagaacac tttcagaaaa atggggcaca gactataatc attagtataa 3720

gaagtagact gtatttatcg cttctacttt gaaaatattg ttcactggga tatatttgtt 3780

cagaaatcat tattgttttc tggacttgcc ttgtgatgag aagacttcaa tgtccatttc 3840

cttttatgga ttaatgttca ttttacacat atattttgcc atagtgtgtc ttagatagta 3900

aatccttaaa atgcaaagtt caaacttact atgatttatt taattccttt agagcaaggt 3960

ggacacagtt aaggtattta cttaactcct aaagtctctg ctctcataga gtttttattc 4020

cactagaaat agagatacaa tgaacaacaa caaaaaaaca tatcacatgg tgacaagtga 4080

tgtgaagaaa aat 4093

<210> 3
<211> 4154
<212> DNA
<213> Homo sapiens

<400> 3

```
caccccaccc cgcccaccaa gtcttacagc taaaggaagg tcctactgac attcattgaa      60
agaatacagg ctttcctaat acccacgacg tgcactatga cccagctccc tacagtcact     120
gccaggtgac ctcctacggc gcttggagac ctgagcagcc ttgcactctt aggcggcagc     180
gtcctttctc cctcttgcca gtaactgctg ccatgtcaac cagtggaatc aagggcttct     240
ggcgagcagc agctgaaaag ggcgcctctg caggcaggcg gatcactttc tataagttta     300
agctacaggc tgagaccttt tattctgtac gagaaaggat cctttactgt cccacgcact     360
agattcttct actttacgtg taacttttca aaactttatt agtttagtta ctgaactgcc     420
tccatggggt cctaattcca ggctcagaat cttccatcca gccttctaaa actctacctt     480
caaaagaggt caaaaagact gtgtaatgac cttgtaaaac actaacccca gacagtggaa     540
ccaatcctga tctcttaaac ccccagtgag tcataagagc ttgagaagat ttcaaggaaa     600
tcttccgcac attggacacg gtccaatcag gttctgaaca attcaatcat cccccggtct     660
gtggccacac tcccccaata gatccaaact gaagtaccat ctgggggagg ggtggtttga     720
ggcagggagt agagaaagca tggagtggta ggtgggggtg gggtgggggt tcctctgggt     780
tctctcggtt caggaggtcc aaagaagacg aagcagccca gaggatttgc ggctgcaggt     840
tctgtagtgg agggaaggtg ggcggacagg tctcacccag ctactactgg agggggagaa     900
tgggagggca gcagccaggg gacaaaggtg ggacaagagg ctctgtaatc atctctcggg     960
caggagaagg actgggcgct ggagtggcga gggggtctgg ggagctcact ccctgcagaa    1020
gacgtactcg gtgtagctgg tccagatctt gtcctcgctc tggtcggtgc tgctggcaaa    1080
ggcgcaggtg cccgtggagc tgcacgccac catgtggaag cccgactcgg acagcttgtc    1140
gaaggcctgc tccaggaagt tgaacttgag gtaatagcgc gaggtgtagc gctccggggg    1200
acggtcgggg tcccggcttt cgttcagggt gtccccaaac acctccttgg ccagcgacgt    1260
ctttccgcaa acggtgatgc gcgccactcg ccggaacttg gcgtccgcct gcgcgtcccg    1320
cccgatggtg taggagccgc ggtagccgat ggtgatgtag cccgagcgcc ggctgccgtc    1380
cagcgactgg gacggcgtga gcagcgggcc cgccgcgccc ccggacggac tgcggctagc    1440
cagctccagc gtgggcgacg gcgccccggc agaggcgccc tcctgctgtt cgggctccga    1500
gtagccaagc ggcagcagct cgtcacccag cgagccctcc ttgtgcaccc cgcgccgcga    1560
gggcggcggc cccgggccgg gctgctgggg cgcccccgagg cggcgcacga gctctggcag    1620
ctcgaagtac tcggcctcgc gctgcagccg gctgcgctcg gggaagtagt cgggcagcac    1680
gagctgcaag tcccgcaggt aatccaggat gtagcggaag aggaagccgt cccggtccag    1740
aaagaagcgg cctttgctgt cccgggccag ctcctgcggc tgctgctgcg tgaacatgcg    1800
ccagagcagc gagtcgggca ccgacaccac cgtgcagcgc cgggtcacgt acacctggcc    1860
ccccacgttc agctccacga tgtcggggaa gagcggtggc tccgcggagg acgacgagga    1920
```

gccactgccg cccccgccgc cgcccccgtt gggtaatcca cgtgtgctgt ccgccagagc 1980
catgacagag aggtggccgg gccgggacag tggcaggaag ccgcgctgca ctcaggagct 2040
gcaaccgcct tccccggagc cccggaaccc ggacgctcgc tcagccctgc gccccgccgc 2100
cgccgccgcc gccaccgccg ccaccgccac cgccgccacc tcctagagcc gcgcggagcc 2160
gagcggtgcg agcgcgccgc tgtgcgcccc cttgagttcc agtgcgctcc gcccgccttg 2220
cccgcgcgct ccaggcgagt gccgggtcgc ggcccgcgcg ctgcttaagt agagccgcct 2280
gctctggtgc cgtggcgcgc aggaggcgtg ggcggcggcg gagcctcgct taccgggcga 2340
ggggaggtgg ggagcgggga ctcgatttgc atcttaaacg cggacgccac gcggggctgc 2400
tggctccacc ccggctcgcg agcgctgggg gcgcccggac tcccacccgc tccccaccca 2460
ccagccccgc cccgtggtcc cgcccaactt tctccctctt ccgaagagga cccgcccgag 2520
acttggcggc ttcggaagac agctccttgg agggacagtc accgcgcccc ctcccctctc 2580
gccccagccc gcacccaagg actctggaag gaggacgtga actttcggaa gcctgtcgcc 2640
ccgggagtgg aaatgcgtgg ccgcctccgg ccctcccgcc tgcggaactg ggcgcttcca 2700
gcagtgcagc tgcggcctgc catcgttggt tgtcgtgtca ctgaaagtag agagacgcga 2760
aaaacctgga gagacgaaag agccgcccca gtccgagcgc agccagattt tgttcaagtt 2820
gccttgcagc cagcctggaa aataaataaa taaacctaga aacaaaaagc aaacctcagt 2880
gtcccgcagt aggatgcttt acgatagttt attttctggc tgcagagaca aaatgagatg 2940
aagcttcaaa tctgctctta attgcaattc cctcttcccg cagggccttc gaggcacttc 3000
acttcgaagt gaaggggtaa gactccggaa cgttcggtct ccgggtccca ttcagccttc 3060
gttgaatcgc gcccttagac tcacaacagt tgcagatcta acgtaatttt taaagccacc 3120
tttggcgcct gcatgtttat ccttccaaaa gtccaggcgc cccttctctc ccgcatgcct 3180
cggtcactct tcggccctgt ccaagttccc gtcagtctca gaaagggata tttatatatc 3240
tgaaggccct tgtctggatc ccatctctca actcctgctg cctacacgtt tgcttttccc 3300
cctcggactg cagcccacag aaccgccccc tccccctgga agctttccct ggcttcccag 3360
gtgggtgggg cctctggatg gctccctaca cactgtctgc gctgctccca gcacgttctg 3420
aaatgtcata tttatgagtg attatctgat cactgatttg ggtatgaaaa taagtaagag 3480
ctcctgaatg ctgcaaaaag agaagagtgc agaggagggt gtcaagaaat gattgggtca 3540
ctgaatgttt cttgtgtacc tgctattatt tgcagggcac cagggagtga ggaatgcaaa 3600
ggcgaataat ctccaaagcg ctttcagtgt agcccaataa gcaaagggtc aggggtctgc 3660
gcagttatca catcagtttg aagtatagac cacacatcag gataaatcag gcaagatctt 3720
tgttcttgat aaaatttatc cttctgtgta actacccagt gagcttcaca gtctcctcac 3780

agataaatgt catttttatt ttgaaatgtt tatatctgct caacaattac cgcgctcatg 3840

tcagtgtgct gaaatgagat caaccccaa accagttatc ttaattgcaa gtacagattt 3900

tgatactgaa tgtatttcaa cattcttttt ttgttgttgt tctgttcttt gggataaata 3960

atagataaca caaaacttcc aagtcccagt gcaggatttc gaggtggttg tgctactagg 4020

cgttatatta gtgtcagaga cctgacaaaa ccagcctctt ttctacaaat ccaagaaaag 4080

ccttctttgt atcagttctg cacatatttt tgtataatta ccattgctca ttgcatcagc 4140

aaatcctgcc agct 4154

<210> 4
<211> 20
<212> DNA
<213> Artificial Sequence

<220>
<223> primer

<400> 4
ggttttgatt agagttgtaa 20

<210> 5
<211> 20
<212> DNA
<213> Artificial Sequence

<220>
<223> primer

<400> 5
caatttcaat caataaaacc 20

<210> 6
<211> 10
<212> DNA
<213> Artificial Sequence

<220>
<223> Tag sequence

<400> 6
aggaagagag 10

<210> 7
<211> 31
<212> DNA
<213> Artificial Sequence

<220>
<223> T7 promoter sequence

<400> 7
cagtaatacg actcactata gggagaaggc t 31

<210> 8
<211> 23
<212> DNA
<213> Artificial Sequence

<220>
<223> primer

<400> 8
aatttttggt tattttggga agc 23

<210> 9
<211> 24
<212> DNA
<213> Artificial Sequence

<220>
<223> primer

<400> 9
ctaatcaaaa cctaaacatc gtcg 24

<210> 10
<211> 25
<212> DNA
<213> Artificial Sequence

<220>
<223> primer

<400> 10
gttttagaaa ggggaataaa aggtc 25

<210> 11
<211> 23
<212> DNA
<213> Artificial Sequence

<220>
<223> primer

<400> 11
ccgacaaacc taaaataaac gta 23

<210> 12
<211> 22
<212> DNA
<213> Artificial Sequence

<220>
<223> primer

<400> 12
gtcgggatag tggtaggaag tc 22

<210> 13
<211> 21
<212> DNA

<213> Artificial Sequence

<220>
<223> primer

<400> 13
gcgcgactct aaaaaataac g 21

<210> 14
<211> 79
<212> DNA
<213> Homo sapiens

<400> 14
ggccctgatt agagctgcaa attccggcgc cgcgtcgcag gaatcgtctt gcaattcccg 60

gccccactga ctgaaactg 79

<210> 15
<211> 33
<212> DNA
<213> Artificial Sequence

<220>
<223> primer

<400> 15
gggatattaa gtggagttat tttggtttta gtt 33

<210> 16
<211> 21
<212> DNA
<213> Artificial Sequence

<220>
<223> primer

<400> 16
ccctcccaac atccttccta a 21

<210> 17
<211> 182
<212> DNA
<213> Homo sapiens

<400> 17
aatctctggt cactttggga agcgcgcgtg tctcctagag gctagtgaga cggtccccgc 60

gtcgtgcctg tgccggcgac acgcggggaa agctccaggg gcgccacctt cccgggggc 120

caggagggaa gggagacgcc cgcagagaag gcgggaggcg acgatgctca ggccctgatt 180

ag 182

<210> 18
<211> 93
<212> DNA
<213> Homo sapiens

<400>  18
gcctcagaaa ggggaacaaa aggccgggcc agccaggctc tgcgcctcac cccgggaggg        60

tccgctgccc cacgcctacc ccaggcctgc cgg                                     93


<210>  19
<211>  154
<212>  DNA
<213>  Homo sapiens

<400>  19
gccgggacag tggcaggaag ccgcgctgca ctcaggagct gcaaccgcct tccccggagc        60

cccggaaccc ggacgctcgc tcagccctgc gccccgccgc cgccgccgcc gccaccgccg       120

ccaccgccac cgccgccacc tcctagagcc gcgc                                   154


<210>  20
<211>  18
<212>  DNA
<213>  Artificial Sequence

<220>
<223>  primer

<400>  20
agcgagcgta tttggttc                                                      18


<210>  21
<211>  21
<212>  DNA
<213>  Artificial Sequence

<220>
<223>  primer

<400>  21
atcctccgaa acaaactaac g                                                  21


<210>  22
<211>  33
<212>  DNA
<213>  Artificial Sequence

<220>
<223>  primer

<400>  22
gggatattaa gtggagttat tttggtttta gtt                                     33


<210>  23
<211>  21
<212>  DNA
<213>  Artificial Sequence

<220>
<223>  primer

```
<400>  23
ccctcccaac atccttccta a                                               21


<210>  24
<211>  100
<212>  DNA
<213>  Homo sapiens

<400>  24
agcgagcgca cctggcccgg cgaccgctgg agctgcgcag cgcgcctcgg agctgcctgc      60

gggcgcacgc cgtcttcccc gccagtctgc cccggaggat                          100
```